# EUROPEAN PATENT SPECIFICATION

(11) **EP 3 713 986 B1**
(45) Date of publication and mention of the grant of the patent: **01.04.2026**
(21) Application number: 18811610.7
(22) Date of filing: 20.11.2018
(51) Int. Cl.: C08G 63/08, C08G 63/90, A61K 9/00, A61K 47/18, A61K 47/20, A61K 47/34, A61K 31/00

(54) **COMPOSITION**
ZUSAMMENSETZUNG
COMPOSITION

(30) Priority: 20.11.2017 GB 201719139
(43) Date of publication of application: 30.09.2020
(73) Proprietor: ReBio Technologies Oy, Raisio 21200 (FI); ReBio Technologies Limited, Hong Kong (HK)
(72) Inventor: GLEN, Jonathan, Hong Kong (CN); JUKARAINEN, Harri, Raisio 21200 (FI); TUOMINEN, Jukka, Horsham, RH12 3GH (GB)
(74) Representative: Gill Jennings & Every LLP
(86) International application number: PCT/GB2018/053349
(87) International publication number: WO 2019/097262

(56) References cited:
- US-A- 5 434 242
- US-A1- 2013 066 042
- US-A1- 2016 158 402
- US-B1- 6 353 030
- YAMINI PANDYA ET AL: "ATRIGEL, IMPLANTS AND CONTROLLED RELEASED DRUG DELIVERY SYSTEM", INTERNATIONAL JOURNAL OF BIOPHARMACEUTICS, 1 January 2014 (2014-01-01), pages 208 - 213, XP055554581, Retrieved from the Internet <URL:http://www.ijbonline.com/article/208-213(ijbonline).pdf>

## Description

### Field of the invention

The present invention relates to bioresorbable polyesters, their methods of purification, and pharmaceutical compositions, preferably in-situ forming implant compositions, comprising such polyesters.

### Background of the invention

Controlled release pharmaceutical compositions are known, and are understood to be compositions designed to maintain the therapeutic dose of an active pharmaceutical ingredient (API) over a specified (and at times a particularly extended) period of time. Bioresorbable polyesters - which are understood to be polyesters that degrade in vivo - are useful in a range of controlled release pharmaceutical compositions. Bioresorbable polyesters degrade in vivo by hydrolysis of their ester backbone into non-toxic products. Such polyesters may be produced by way of a variety of different polymerisation methods, and such methods often make use of a metal catalyst. Following their polymerisation, bioresorbable polyesters may be purified before they are included in a controlled release composition. For example, US 6 461 631 discloses a purification process whereby a polyester is dissolved in dichloromethane (DCM), and then precipitated in methanol. US 6353 030 discloses a purified polylactide, containing one or more metals having a concentration of at most 10 ppm. US 2013/066042 discloses a method for reducing the content of a metal catalyst in a biodegradable and absorbable copolymer, that includes washing the copolymer with a mixed solvent comprising acetic acid and isopropanol. US 2016/158402 discloses a method of preparing compositions of P4HB (poly-4-hydroxybutyrate) with high purity, by washing P4HB biomass prior to solvent extraction and then precipitating P4HB from solution. Lastly, US 5434 242 relates to resorbable homopolymers and copolymers which are substantially free from polymerization catalysts and processes for preparing them.

Examples of bioresorbable polyesters include polylactic acid, polyglycolic acid, polycaprolactone, poly(lactide-co-glycolide), poly(lactide-co-caprolactone), poly(glycolide-co-caprolactone), and poly(lactide-co-glycolide-co-caprolactone), all of which may be produced by ring-opening polymerisation.

Controlled release pharmaceutical compositions can have various advantages. For example, by maintaining the therapeutic dose of an API over an optimal period of time, the dose frequency may be lowered and drug efficacy may be improved. However, achieving controlled release of an API can be problematic, and as such, there are a number of problems associated with existing controlled release pharmaceutical compositions. Ideally, the initial amount of drug released from a controlled release pharmaceutical compositions corresponds to the therapeutic dose, where this dose is then maintained by the controlled release pharmaceutical composition. However, one problem associated with existing controlled release compositions is when there is no (or no appreciable) release of the drug for an unduly long time period following administration of the composition (the time period being referred to as the "lag time"). Another problem is when an excessively high initial burst of the drug is released from the composition - also referred to as the "burst effect". This initially excessive release can cause a number of undesirable drug-related side effects. A further problem is when, following this initial burst release, there is a rapid drop in release rate, rather than a sustained and steady release of the drug at the desired therapeutic dose.

Examples of existing controlled release pharmaceutical compositions are those that include polymeric microparticles as drug carriers. Such polymeric microparticles are substantially spherical microparticles (also referred to as microspheres) that encapsulate drug molecules. The polymeric microparticles often include a bioresorbable polyester. The polymeric microparticles may protect unstable drugs in vivo, release the drug over a prolonged period of time, and achieve a more targeted drug delivery thereby minimising undesirable systemic effects. However, existing polymeric microparticles formulations typically suffer from a high initial burst effect, a long lag time, such that a steady drug release is not maintained.

Other examples of existing controlled release pharmaceutical compositions are those that function as an in-situ forming implant. One such prior art drug delivery system is the Atrigel system, which includes a bioresorbable polyester, and can be used for both parenteral and site-specific drug delivery (Pandya. Y. et al., International Journal of Biopharmaceutics. 2014; 5(3): 208-213.). In the Atrigel drug delivery system, the polymer component and the API are dissolved in a biocompatible solvent. This solution of polymer and API is then administered by injection, after which it solidifies in vivo upon contact with aqueous bodily fluids and forms a solid implant. Such in-situ forming implants may result in targeted drug delivery to a particular area and achieve some level of sustained release. However, drug delivery systems such as these still suffer from a high initial burst effect. Another in-situ forming implant including a bioresorbable polyester where the burst effect may be improved is disclosed in WO2014001904, where the in-situ forming implant is administered by subcutaneous injection.

### Summary of the invention

In a first aspect, the present invention provides a controlled release pharmaceutical composition comprising at least one active pharmaceutical ingredient in a biocompatible solvent and at least one polyester, wherein the pharmaceutical composition is in the form of an in-situ forming implant composition, wherein the biocompatible solvent is selected from N-methyl-2-pyrrolidone, triacetin, dimethylsulfoxide, benzyl benzoate, glycofurol or combinations thereof, wherein the polyester is a bioresorbable polyester and wherein the metal content of the polyester is > 0 ppm and < 40ppm.

In a second aspect, the present invention provides a method of forming a controlled release pharmaceutical composition, wherein the pharmaceutical composition is in the form of an in-situ forming implant composition, comprising purifying a bioresorbable polyester with a metal content of > 0 ppm comprising the steps of:
i) dissolving the polyester in an organic solvent to form a polyester-solvent solution, wherein the organic solvent comprises at least one heteroatom selected from oxygen, nitrogen, sulphur and phosphorus;
ii) precipitating the polyester from the polyester-solvent solution by combining the polyester-solvent solution with an organic non-solvent, said non-solvent being an alcohol; and
(iii) separating the precipitated polyester from the solvent and non-solvent to obtain a purified polyester;
   such that the metal content of the purified polyester is > 0 ppm and < 40ppm; and
(iv)combining the purified polyester with at least one active pharmaceutical ingredient in a biocompatible solvent;
to form the controlled release composition, wherein the biocompatible solvent is selected from N-methyl-2-pyrrolidone, triacetin, dimethylsulfoxide, benzyl benzoate, glycofurol or combinations thereof.

In a third aspect, the present invention provides a kit for injecting a subject with a controlled release pharmaceutical composition, wherein the pharmaceutical composition is in the form of an in-situ forming implant composition, comprising, a first container comprising at least one bioresorbable polyester according to the first aspect, and a biocompatible solvent selected from N-methyl-2-pyrrolidone, triacetin, dimethylsulfoxide, benzyl benzoate, glycofurol or combinations thereof; a second container comprising at least one active pharmaceutical ingredient; and a needle capable of attachment to at least one of the first or the second container.

It has been surprisingly found that a polyester that has a reduced metal content in comparison to the prior art achieves improved controlled release when the polyester is included in a pharmaceutical composition. When a metal catalyst is used during the formation of a polyester, residual metal catalyst remains in the polyester following its formation. In the prior art, the residual metal catalyst that remains in the polyester is typically above 40ppm, by virtue of the amounts of metal catalyst used during commercial production of bioresorbable polyesters. Without wishing to be bound by theory, it is thought that this residual metal catalyst triggers degradation of the polyester, and also of the API of the pharmaceutical formulation, where this polyester/API degradation adversely impacts the release profile of the eventual controlled release formulation. It is thought that the particularly low metal catalyst content of the polyesters disclosed herein provides a reduction in the burst effect, reduced lag time, improved API stability, and thus prolonged sustained and steady release, when these polyesters are included in a controlled release pharmaceutical composition. The controlled release pharmaceutical composition may typically be administered to humans but may also be administered to other members of the Animalia Kingdom, e.g. other mammals, fishes, birds, or reptiles.

The particularly low metal content of the polyesters disclosed herein is achieved by way of a new purification method. The method has applicability to polyesters that have a non-zero metal content prior to purification (i.e. a metal content > 0 ppm) e.g. when a metal catalyst has been used during the formation of the polyester. During step i) of the method, the polyester is dissolved in an organic solvent wherein the organic solvent comprises at least one heteroatom selected from oxygen, nitrogen, sulphur and phosphorus. Without wishing to be bound by theory, it is thought that the heteroatom of such organic solvents coordinates with the metal present in the polyester. It is thought that this coordination allows the organic solvent to form a stronger interaction with the metal than the interaction between the polyester and the metal, such that the metal is separated from the polyester during the precipitation step (step ii) and the separation step (step iii). This results in a greater reduction in the metal content in comparison to prior art purification methods.

### Description of the preferred embodiments

As used herein, the term "bioresorbable polyester" refers to the ability of the polyester to degrade in vivo such that it is capable of removal by cellular activity and/or dissolution in a biological system, e.g. in the human body.

As used herein, the term "polyester" takes its usual definition in the art and so refers to a polymer whose polymer backbone includes a plurality of ester functional groups. As used herein, the term "polymer" takes its usual definition the art and so refers to a homopolymer or copolymer formed from the polymerisation of one or more monomers. As such, this term covers stereopolymers, linear polymers, branched polymers (including star-shaped polymers, which consist of three or more linear polymers connected to a central core), crosslinked and uncrosslinked polymers. Preferably, the polyester is a co-polymer.

As used herein, the term "homopolymer" takes its usual definition in the art, and so refers to a polymer whose polymer chains comprise one type of monomer. As used herein, the term "co-polymer" takes its usual definition in the art, and so refers to a polymer whose polymer chains comprise two or more different types of monomers. The skilled person will appreciate therefore that the term "co-polymer" encompasses polymers that include three different types of monomers (which can at times be referred to in the art specifically as "terpolymers"). The term "block co-polymer" takes its usual definition in the art and so refers to a copolymer whose polymer chains include two or more blocks of monomers. Each block is comprised of a particular monomer type, where at least two of the blocks present comprise a different monomer type to one another. A di-block co-polymer, a tri-block copolymer, and a tetra-block copolymer, each refer to copolymers with two, three, and four monomer blocks respectively.

As used herein, the term "monomer" takes its usual definition in the art and so refers to a molecular compound that may chemically bind to another monomer to form a polymer. The one or more monomers that form the polyester polymer disclosed herein are understood to include all enantiomers, diastereomers, racemates and mixtures thereof of the monomers in question. Consequently, unless expressly stated to the contrary, the polyesters disclosed herein are understood to include all possible stereoisomers.

The skilled person will be familiar with the conditions required to form the polyester. Preferably, the polyester is formed from the polymerisation of one or more cyclic monomers by ring opening polymerisation. Typically, polymerisation is carried out using an initiator in the presence of a catalyst.

The specific bioresorbable polyester chosen will depend on the desired properties of the eventual pharmaceutical formulation. There are numerous examples of bioresorbable polyesters. Examples of bioresorbable polyesters include polyesters formed from the polymerisation of lactide, glycolide, caprolactone, tetramethylglycolide, trimethylene carbonate, p-dioxanone, gamma-Valerolactone, delta-Valerolactone, 1,4-dioxane-2,5-dione, or combinations thereof. Other examples of polyesters include polydihydropyrans, or polyhydroxyalkanoates, such as polyhydroxybutyrates (PHB); PHB/b-hydroxyvalerate copolymers (PHB/PHV); poly-b-hydroxypropionate (PHPA).

Preferably, the polyester is formed from the polymerisation of lactide, glycolide, caprolactone, or combinations thereof. Such monomers are cyclic and are often used to form polymers by way of ring opening polymerisation. More preferably, the polyester is formed from the polymerisation of two or more of lactide, glycolide and caprolactone

Preferably, the polyester is polylactic acid, polyglycolic acid, polycaprolactone, poly(lactide-co-glycolide), poly(lactide-co-caprolactone), or poly(glycolide-co-caprolactone), poly(lactide-co-glycolide-co-caprolactone, more preferably poly(lactide-co-glycolide), poly(lactide-co-caprolactone), poly(glycolide-co-caprolactone), or poly(lactide-co-glycolide-co-caprolactone), inclusive of all possible stereoisomers of these polymers.

The polyester may be capped with polyethylene glycol (PEG), polypropylene glycol (PPG), and/or polyvinyl alcohol (PVA), at the terminus of one or more polymer chains. The polyester may also be a block co-polymer, comprising (in addition to one or more polyester blocks) at least one block of polyethylene glycol (PEG), polypropylene glycol (PPG), or polyvinyl alcohol (PVA).

The metal content of the purified polyester is > 0 ppm and < 40ppm. Preferably, the metal content of the purified polyester may be < 35ppm, < 30ppm, < 25 ppm, < 20 ppm, < 15 ppm, < 10 ppm, < 5 ppm, or < 2 ppm.

As the skilled person will appreciate, a metal catalyst is a metal that accelerates and/or initiates the polymerisation reaction. As such, the term "metal catalyst" covers elemental metals, inorganic metal compounds, metal salts, oxides, halides, hydroxides, carboxylates, organometallic compounds, metal complexes metallocenes. As used herein, the ppm (i.e. parts per million) content of the bioresorbable polyester refers to the mass of elemental metal present in relation to the total mass of the polyester, e.g. a value of 1 ppm refers to 1 × 10⁻⁶ grams of elemental metal per 1 gram of polyester. The metal content refers to the total amount of metal present in the polyester, inclusive of free metal, metal weakly coordinated to the polymer, plus metal strongly coordinated or bonded to the polymer. The metal content is measured by inductively coupled plasma atomic emission spectroscopy, which is a method familiar to those skilled in the art, and can be carried out using an Agilent MP-AES 4200 with microwave digestion (the acronym "MP-AES" refers to microwave Plasma-Atomic Emission Spectrometry), with results calibrated by way of metal standards, measurement of a blank sample, and measurement of a sample spiking/recovery test with a known amount of metal. The type of metal present in the polyester will correspond with the metal used as a catalyst during the formation of the polyester. For example, the metal is selected from tin, zinc, iron, aluminium, titanium, platinum, bismuth, manganese, antimony, nickel, calcium, magnesium, sodium, lithium, yttrium, lanthanum, samarium, zirconium, ruthenium or combinations thereof.

Disclosed herein is a controlled release pharmaceutical composition comprising at least one active pharmaceutical ingredient and at least one of the purified polyester(s) disclosed herein. The one or more active pharmaceutical ingredients comprised by the compositions disclosed herein are understood to include all enantiomers, diastereomers, racemates and mixtures thereof. The drug can also be a salt, a solvate, a hydrate, a pro-drug, a co-crystal, a derivative, in free base form, or a mixture thereof.

The active pharmaceutical ingredient comprised by the compositions disclosed herein may include one or more H2 receptor antagonists, antimuscarinics, prostaglandin analogues, non-steroidal anti-inflammatory agents, proton pump inhibitors, aminosalycilates, corticosteroids, chelating agents, cardiac glycosides, phosphodiesterase inhibitors, thiazide, diuretics, anesthetic agents, carbonic anhydrase inhibitors, antihypertensives, anti-cancers, anti-depressants, calcium channel blockers, analgesics, opioid antagonists, antiplatels, anticoagulants, fibrinolytics, statins, adrenoceptor agonists, beta blockers, antihistamines, respiratory stimulants, micolytics, expectorants, barbiturates, anxiolytics, central nervous system agents, tricyclic antidepressants, 5HT1 antagonists, opiates, 5HT1 agonists, antiemetics, antiepileptics, dopaminergics, antibiotics, antifungals, anthelmintics, antivirals, antiprotozoals, antidiabetics, insulin and its derivatives, GLP-1 agonists, thyrotoxins, female sex hormones, male sex hormones, antioestrogens, hypothalamics, pituitary hormones, posterior pituitary hormone antagonists, peptide drugs, protein drugs, protein kinases, antigens, antidiuretic hormone antagonists, bisphosphonates, dopamine receptor stimulants, androgens, steroid reductase inhibitors, non-steroidal anti-inflammatories, immuno suppressants, local anaesthetic, sedatives, anti-psoriatics, silver salts, topical antibacterials, vaccines, or vaccine antigens.

The active pharmaceutical ingredient comprised by the compositions disclosed herein may include one or more central nervous system agents. Central nervous system agents include antipsychotic drugs e.g. risperidone, haloperidol, olanzapine, quetiapine, ziprasidone, aripiprazole, paliperidone, lurasidone; selective serotonin reuptake inhibitors e.g. fluoxetine, citalopram, sertraline, paroxetine, escitalopram; benzodiazepines e.g. clonazepam, alprazolam, lorazepam; or, mood stabilizers e.g. carbamazepine, lamotrigine, oxcarbazepine.

Preferably, the active pharmaceutical ingredient includes one or more GLP-1 receptor agonists. GLP-1 receptor agonists are particularly desirable to include in a controlled release composition. GLP-1 receptor agonists include exenatide, liraglutide, lixisenatide, albiglutide, dulaglutide, taspoglutide, semaglutide.

The molecular weight of the active pharmaceutical ingredient can be at least 100g/mol, at least 1000g/mol, or at least 1200g/mol. The molecular weight of the active pharmaceutical ingredient can be at most 250,000g/mol, 80,000g/mol, or 20,000g/mol. Preferably, the molecular weight of the active pharmaceutical ingredient is at least 200-500g/mol.

The bioresorbable polyesters of the present invention can be applied to a wide range of drug delivery uses. The purified polyesters may be used as a matrix for drug delivery systems e.g. solid implants, in-situ forming implants, micro and nanoparticles, micelles, patches, buccal inserts, vaginal inserts, intrauterine systems, cervical systems, suppositories, root canal filling, contact lenses, as well as devices used for supporting tissue or bone healing or regeneration.

The controlled release pharmaceutical composition is in the form of an in-situ forming implant composition. Such formulations are particularly desirable as long-term controlled release compositions.

The composition comprises at least one purified polyester disclosed herein and at least one active pharmaceutical ingredient in a biocompatible solvent. The term "biocompatible solvent" takes its usual definition in the art and so refers to a solvent that is not harmful or toxic to living tissue. The biocompatible solvent is a liquid capable of dissolving at least 1 mg/ml of the purified polyester at 37 °C, and capable of dissolving or dispersing the at least one active pharmaceutical ingredient. The biocompatible solvent is selected from N-methyl-2-pyrrolidone, triacetin, dimethylsulfoxide, benzyl benzoate, glycofurol or combinations thereof.

Once administered, the controlled release pharmaceutical composition may release the pharmaceutically active ingredient over the course of 1 day to 5 years, or from 1 week to 1 year. The composition may be bioresorbed over such a period of time as from 1 week to 5 years, or from 1 month to 2 years.

The controlled release pharmaceutical composition may be used to treat conditions such as type 1 and type 2 diabetes, CNS diseases, pain, ophthalmic diseases, autoimmune diseases, chronic infections, chronic inflammations, and cancer.

Related to, but not in accordance with, the invention is a method of purifying a bioresorbable polyester with a metal content > 0 ppm comprising the steps of:
i) dissolving the polyester in an organic solvent to form a polyester-solvent solution, wherein the organic solvent comprises at least one heteroatom selected from oxygen, nitrogen, sulphur and phosphorus;
ii) precipitating the polyester from the polyester-solvent solution by combining the polyester-solvent solution with a non-solvent, said non-solvent being an alcohol;
   and
(iii) separating the precipitated polyester from the solvent and non-solvent to obtain a purified polyester.

The purified polyester has a particularly reduced metal content in comparison to polyesters purified by way of prior art methods. Further, the purified polyester also has reduced non-polymerised monomer residuals and/or acidic residuals, which are also thought to otherwise induce uncontrolled degradation of the polyester.

Both the solvent used in step i) and the non-solvent used in step ii) are liquids. As used herein, the term "organic" is understood to refer to a compound that includes carbon as part of its structure.

The organic solvent is capable of dissolving at least 1 mg/ml of the polyester at 37 °C. As such, during step i), an amount of at least 1 mg, at least 10 mg, at least 50 mg, at least 100 mg, at least 150 mg, or at least 200 mg, of the unpurified polyester may be added per 1ml of organic solvent.

Preferably, the organic solvent comprises at least one heteroatom selected from oxygen and nitrogen, optionally comprising at least one further heteroatom selected from oxygen, nitrogen, sulphur and phosphorus. More preferably, the organic solvent comprises at least two heteroatoms.

Preferably, the organic solvent has a dielectric constant of at least 10. As will be appreciated by the skilled person, the dielectric constant refers to the ratio of the permittivity of a substance to the permittivity of a vacuum. The dielectric constant of a solvent is a measure of the polarity of a solvent. The dielectric constant can be measured using the method disclosed in CRC, Handbook of Chemistry and Physics (92nd Edition, 2011-2012, chapter entitled "Laboratory solvents and other liquid reagents"). A greater dielectric constant indicates a greater polarity (which can also be referred to as a greater dipole moment). Without wishing to be bound by theory, it is thought that a dielectric constant of at least 10 allows particularly good solvation of the metal in the polyester, allowing a greater amount of metal to be separated from the polyester. More preferably, the organic solvent has a dielectric constant of at least 15, more preferably at least 20, most preferably 22.

Preferably, the organic solvent is selected from acetonitrile, N-methyl-2-pyrrolidone, triacetin, nitromethane, dimethylformamide, dimethylacetamide, dimethylsulfoxide, hexamethylphosphoramide, tetrahydrofuran, or combinations thereof. More preferably, the organic solvent is dimethylsulfoxide. Dimethylsulfoxide as the organic solvent provides particularly good results, as can be seen from the examples. It is thought that this is due to dimethylsulfoxide having a particularly high dielectric constant.

After step i) has been completed, it is possible to allow the polyester-solvent solution to stand before the commencement of step ii). Preferably, the polyester-solvent solution is allowed to stand for a time period of at least 30 minutes, at least 1 hour, at least 5 hours, at least 10 hours, or at least 15 hours. Without wishing to be bound by theory, it is thought that, for a given temperature, increasing the period of time that the polyester-solvent solution is allowed to stand prior to the commencement of step ii), allows a greater amount of metal to be removed from the polyester.

The method of purification may be carried out at ambient pressure, i.e. a pressure of 1 bar. The skilled person will be familiar with the appropriate temperatures at which the method disclosed herein should be carried out. For example, step (i) may be carried out at 19-189 °C, preferably 21-100 °C, more preferably 30-80 °C, at a pressure of 1 bar. The preferable temperature ranges for step (i) allow further improvement to the removal of metal catalyst.

The non-solvent is capable of precipitating the polyester from the polyester-solvent solution upon addition to the polyester-solvent solution. The capabilities of the non-solvent are such that the non-solvent dissolves less than 1 mg/ml of polyester at 25 °C.

Preferably, during step ii) at least 1 ml of non-solvent is used for every 100 ml of the polyester-solvent solution. As such, during step ii), at least 20 parts, at least 30 parts, at least 40 parts, at least 50 parts, at least 60 parts, or at least 70 parts of non-solvent may be used for every 20 parts of polyester-solvent solution.

The non-solvent may be a primary alcohol, preferably a tertiary alcohol, more preferably a secondary alcohol.

Preferably, the non-solvent is selected from methanol, ethanol, propanol, butanol, pentanol, hexanol, or their combinations thereof. The terms ethanol, ethanol, propanol, butanol, pentanol, and hexanol, are understood to cover all isomers of these compounds. More preferably, the non-solvent is propanol, more preferably isopropanol - this achieves particularly good results, as can be seen in the examples.

Preferably, steps i) and ii) are substantially free of water and/or acid (both organic and inorganic), as the presence of water and/or acid may trigger hydrolysis of the polyester. In one embodiment, steps i) and ii) are completely free of water and/or acid (both organic and inorganic).

Following the separation step iii), the method may be repeated. I.e., following the separation of the precipitated polyester from the solvent and non-solvent, steps i), ii) and iii) may be repeated. Repeated the method disclosed herein results in an even greater reduction in the metal content.

During step iii), the precipitated polyester is separated from the solvent and non-solvent to obtain a purified polyester. This separation step may be carried out by drying, evaporation, filtration, centrifugation, gravitational sedimentation, or a combination of these separation methods. When the separation step is carried out by drying or evaporation, this may be by vacuum drying or evaporation, freeze drying or evaporation, spray drying or evaporation, or, fluidized bed drying or evaporation with or without heat. The skilled person will appreciate that the term "drying" generally refers to the removal of water whilst the term "evaporation" covers the removal of both aqueous and non-aqueous solvents.

The purified bioresorbable polyester may be combined with at least one active pharmaceutical ingredient to form the controlled release composition. Consequently, disclosed herein is a method of forming a controlled release pharmaceutical composition, comprising purifying a bioresorbable polyester with a metal content of > 0 ppm by way of the purification method disclosed herein, and combining the purified polyester with at least one active pharmaceutical ingredient and at least one biocompatible solvent selected from N-methyl-2-pyrrolidone, triacetin, dimethylsulfoxide, benzyl benzoate, glycofurol or combinations thereof, to form an in-situ forming implant composition. The at least one active pharmaceutical ingredient may be one or more of any active pharmaceutical ingredient disclosed herein.

The bioresorbable polyester may be provided as part of a kit for injecting a subject with a controlled release pharmaceutical composition. The kit facilities the preparation of the controlled release pharmaceutical composition to be ready for the injection. The kit comprises a first container comprising at least one bioresorbable polyester as described herein, and a biocompatible solvent; a second container comprising at least one active pharmaceutical ingredient; and a needle capable of attachment to at least one of the first of the second container.

The possible and preferable features associated with the bioresorbable polyester within the kit are as described previously.

The kit may further comprise instructions for use, wherein the instructions for use require the mixing of the contents of the first and second container, following by the injection of the resulting mixture into a subject.

The kit may further comprise a means for mixing the contents of the first container with the contents of the second container, and a means for agitating the resulting mixture.

In one embodiment, the first and second containers are provided as separate parts that are capable of being connected together. In this arrangement, the first and second containers may be provided as syringes that are capable of being connected together in order to mix the contents of the first and second containers.

In another embodiment, the first and second containers are provided as two integrally formed compartments. In this arrangement, the first and second containers may be provided as compartments of a pen, within which the contents of the compartments may be mixed.

The active pharmaceutical ingredient may be provided as a desiccated powder in the kit.

The possible and preferable biocompatible solvents, and the possible and preferable active pharmaceutical ingredient(s), are as described previously.

The following non-limiting examples illustrate the invention.

### Example 1

This example compares the metal contents of a crude polyester, a polyester purified by the method of the present invention, and a polyester purified by a method of the prior art. The metal content was measured by MP-AES (microwave Plasma-Atomic Emission Spectrometry). This example illustrates how the method of the present invention reduces the metal content of a polyester to a greater extent in comparison to prior art purification methods.

A poly(lactic-co-glycolic acid) co-polymer (PLGA) with a non-zero tin content was synthesised internally by ReBio. The tin (i.e. Sn) content of this crude polymer was measured without subjecting the polymer to any purification process. The results are shown as sample 1 in the table below.

A sample of the crude PLGA was then purified using the method of the present invention. Specifically, 2g of the crude PLGA was dissolved in 10 ml of dimethyl sulfoxide (DMSO) to form a 20% w/v polyester-solvent solution. The PLGA was then precipitated by adding 20 parts of the polyester-solvent solution to 80 parts of isopropanol (also called isopropyl alcohol or "IPA"). The PLGA was separated from the DMSO and IPA by gravitational sedimentation and subsequent vacuum evaporation at 55 °C. The tin content of the purified PLGA is shown as sample 3 in the table below.

Another sample of the crude PLGA was purified using a method of the prior art. Specifically, 2g of the crude PLGA was dissolved in 10 ml of dichloromethane (DCM) to form a 20% w/v polyester-solvent solution. The PLGA was then precipitated by adding 20 parts of the polyester-solvent solution to 80 parts of petroleum ether (PeEt). The PLGA was separated from the DCM and PeEt by gravitational sedimentation. The tin content of the purified PLGA is shown as sample 2 in the table below.

As can be seen from the table below (where SD stands for standard deviation) the prior art purification method does not lower the tin content of the PLGA. Meanwhile, the method of the present invention results in significant decrease in the amount of tin.

| **Sample** | **Polymer** | **Purification** | **Sn/ ppm** | **SD** |
|---|---|---|---|---|
| 1 | PLGA5050 | None | 19 | ±1 |
| 2 | PLGA5050 | DCM/PeEt | 21 | +1 |
| 3 | PLGA5050 | DMSO/IPA | 11 | +1 |

### Example 2

This example compares the drug release profiles for a polyester purified by the present invention vs a commercially available crude non-purified polyester. This example illustrates the improved controlled release provided by polyesters purified according to the invention, compared with unpurified polyesters.

A commercially available poly(lactic-co-glycolic acid) co-polymer (PLGA) with a non-zero tin content was obtained (specifically, PLGA5050 polymer ResomerRG502). The tin content of this crude polymer was measured (without subjecting the polymer to any purification process) by MP-AES (microwave Plasma-Atomic Emission Spectrometry), to give a tin content of 42.3 ppm.

A sample of the crude PLGA was then purified using the method of the present invention. Specifically, 2 g of the crude PLGA was dissolved in 10 ml of dimethyl sulfoxide (DMSO) to form a 20% w/v polyester-solvent solution. The PLGA was then precipitated by adding 20 parts of the polyester-solvent solution to 80 parts of isopropanol. The precipitated polymer was then collected and dried under vacuum at 45 C for 2 days. The tin content of this purified polymer was measured by MP-AES (microwave Plasma-Atomic Emission Spectrometry), to give a tin content of 28.4 ppm.

The crude polymer and the purified polymer were each dissolved in DMSO, and an amount of 10 mg/ml of exenatide (a GLP-1 peptide) was then dispersed in each of these solutions.

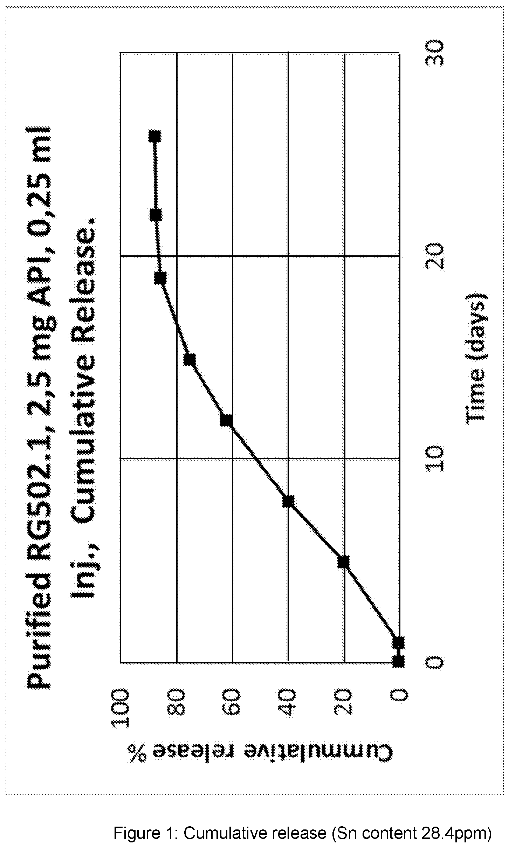

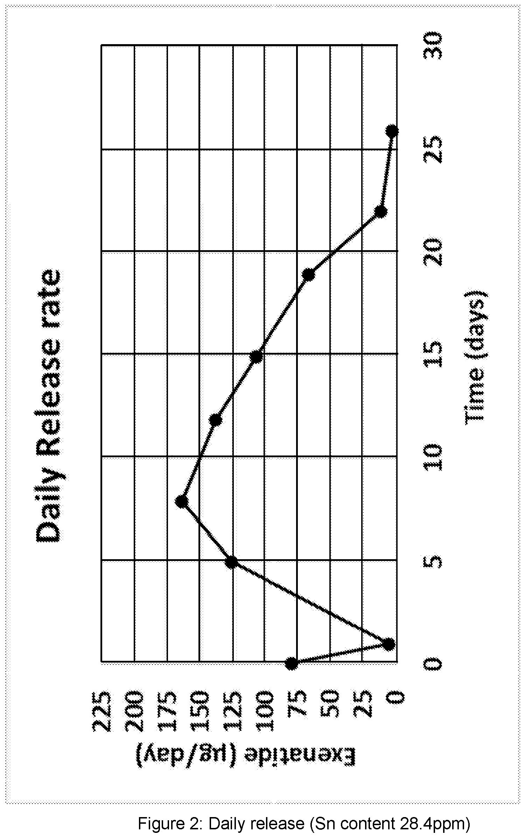

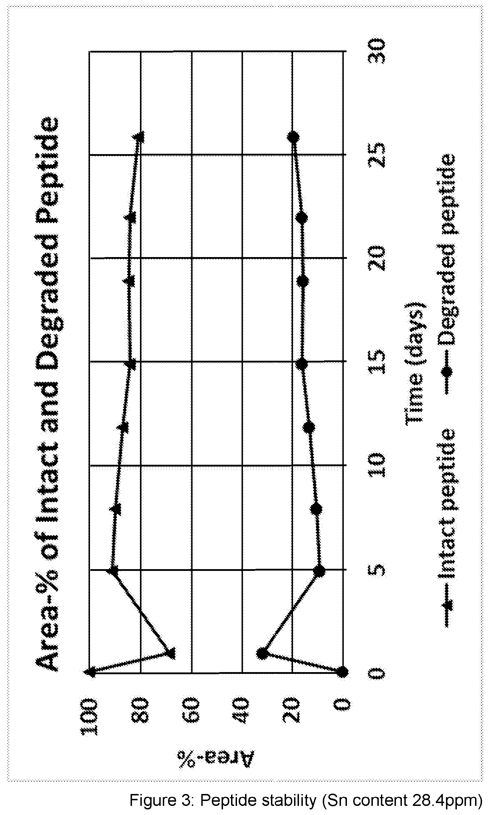

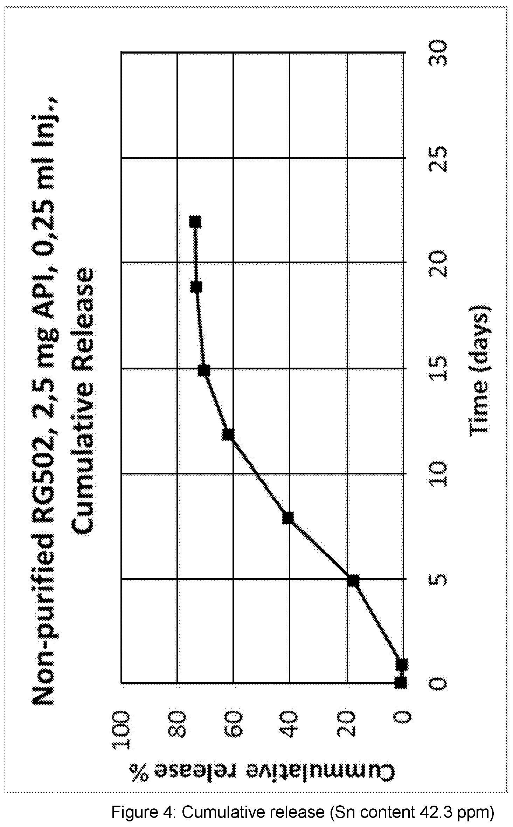

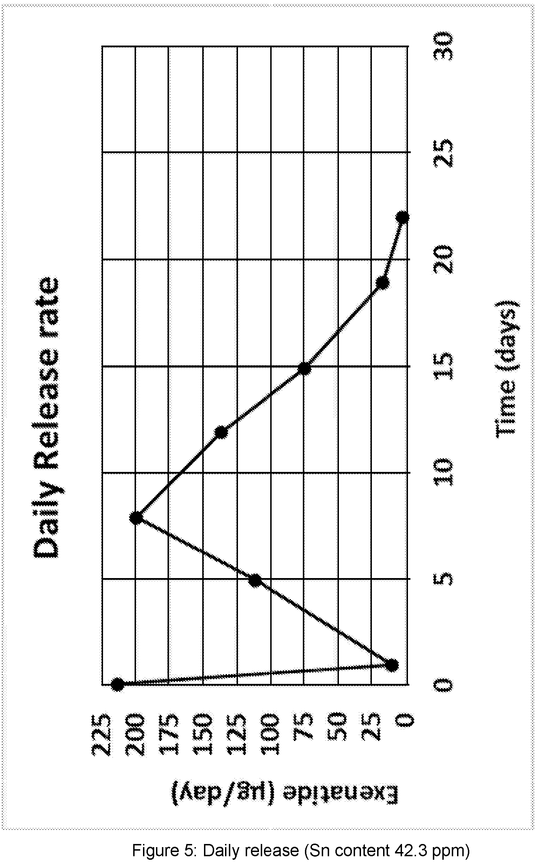

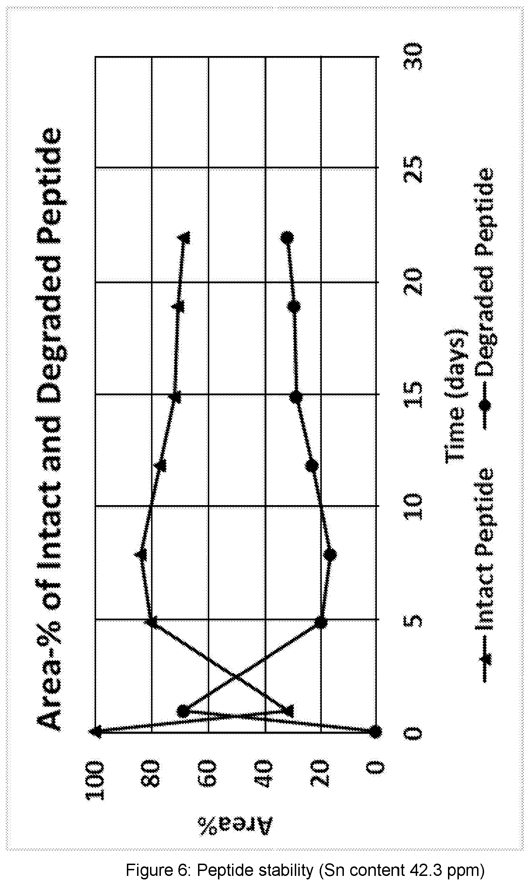

Next the drug release profiles of each solution were measured in phosphate buffered saline (PBS, pH 7.4) by injecting a predetermined amount of each solution in a stainless steel mesh screen pouch, and dropping the pouch in a PBS containing glass vessel, after which precipitation started to occur. The glass vessels were placed in a 37 °C tempered incubator with 60 rpm revolving speed (orbit 2 inches). The in vitro analysis of the peptide release was carried out by sampling the PBS and analysing the intact peptide A in ultra high pressure liquid chromatography equipment (UHPLC). After every sampling the PBS was replaced with fresh PBS solution. The cumulative release profile, daily release and peptide purity graph are shown in figures 1-6 (Figure 1: Cumulative release for an Sn content of 28.4ppm, Figure 2: Daily release for an Sn content of 28.4ppm, Figure 3: Peptide stability for an Sn content of 28.4ppm, Figure 4: Cumulative release for an Sn content of 42.3 ppm, Figure 5: Daily release for an Sn content of 42.3 ppm, Figure 6: Peptide stability for an Sn content of 42.3 ppm). In these figures, RG502 refers to results using crude unpurified polyester, and RG502.1 refers to results using purified polyester. These results show that a polyester purified according to the present invention achieves a higher cumulative release, an longer duration for the daily release rate, and better peptide stability.

### Example 3

A PLGA Polymer (with number average molecular weight of 14800 g/mol) was synthesised internally by ReBio and purified with a prior art method, namely, the polymer was dissolved in dichloromethane and precipitated in petroleum ether, and subsequently dried. The molecular weight of the polymer was then analysed with Gel permeation chromatography (GPC) and the Tin (Sn) amount in the polymer was analysed by MP-AES (microwave Plasma-Atomic Emission Spectrometry).

The polymer was then further purified by dissolving 2g of the polymer in 10 ml of DMSO (20% w/v) and precipitated by adding 20 parts of the polyester-solvent solution to 80 parts of isopropanol. The precipitated polymer was then dried in vacuum at 45 °C for 2 days. The yield in precipitation was measured as 96 %. The molecular weight of the polymer was then analysed with Gel permeation chromatography (GPC) and the Tin (Sn) amount in the polymer was analysed by MP-AES (microwave Plasma-Atomic Emission Spectrometry).

In the following results, PLGA5050 is the polymer purified by DCM/PeEt and PLGA5050D is the polymer that has been further purified by DMSO/IPA.

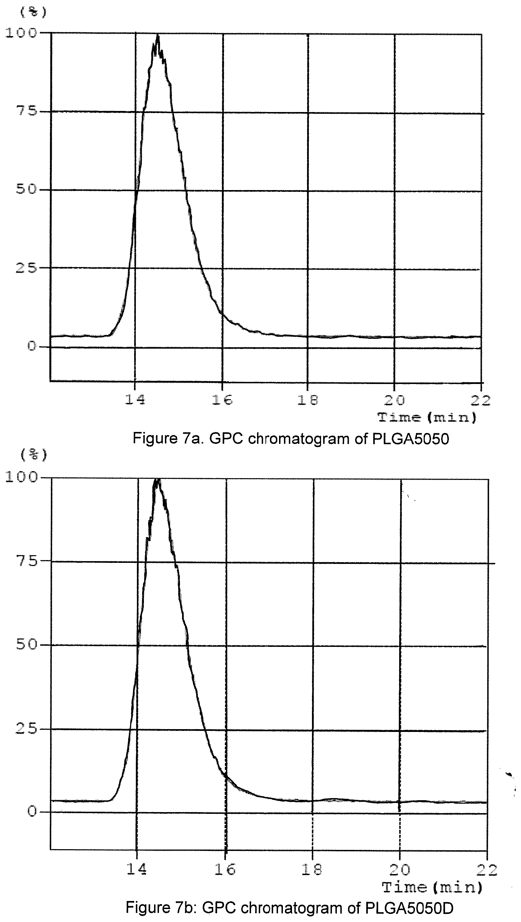

The GPC results are shown in Figure 7a and 7b (GPC chromatograms of the PLGA5050 and PLGA5050D polymer) and in the table below, which show that the purification step does not affect to the molecular weight or polydispersity of the polymer. The tin contents results are also shown in the table below and show that the further purification step provide a reduction in the tin content as the total amount of Sn diminishes by 27.5 % (from 40 ppm to 29 ppm).

| Polymer | Mn (g/mol) | Mw (g/mol) | Sn (ppm) |
|---|---|---|---|
| PLGA5050 | 14800 | 15900 | 40 |
| PLGA5050D | 14800 | 15900 | 29 |

Next, each of PLGA5050 and PLGA5050D were dissolved in DMSO and subsequently the peptide A (peptide A being exenatide) was dispersed throughout each solution.

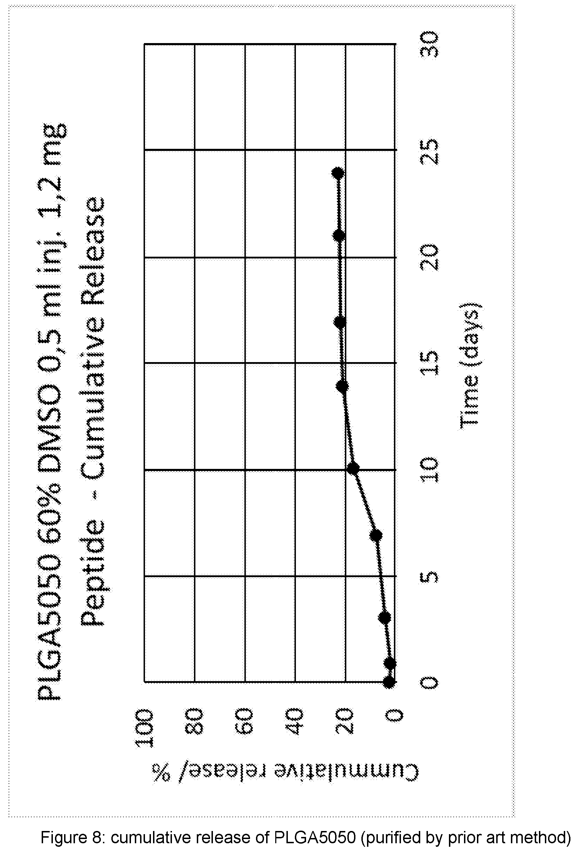

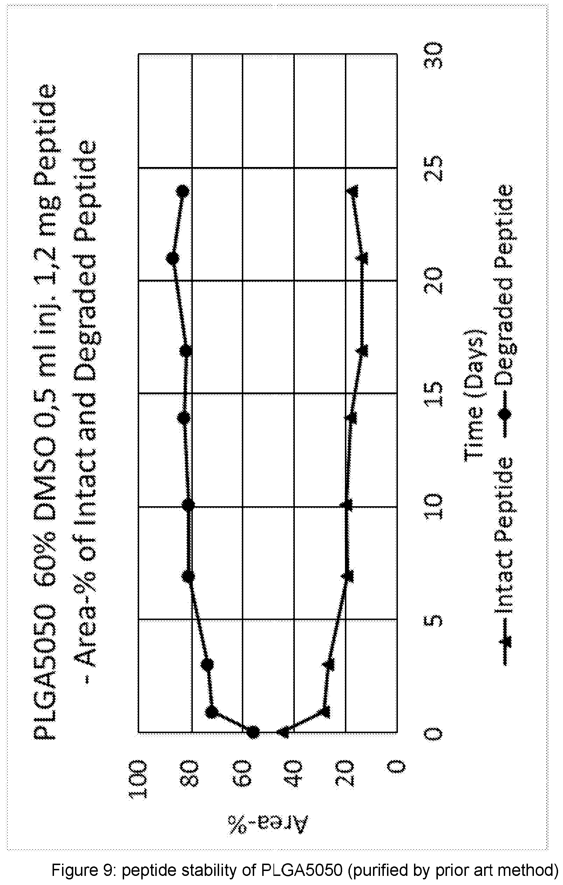

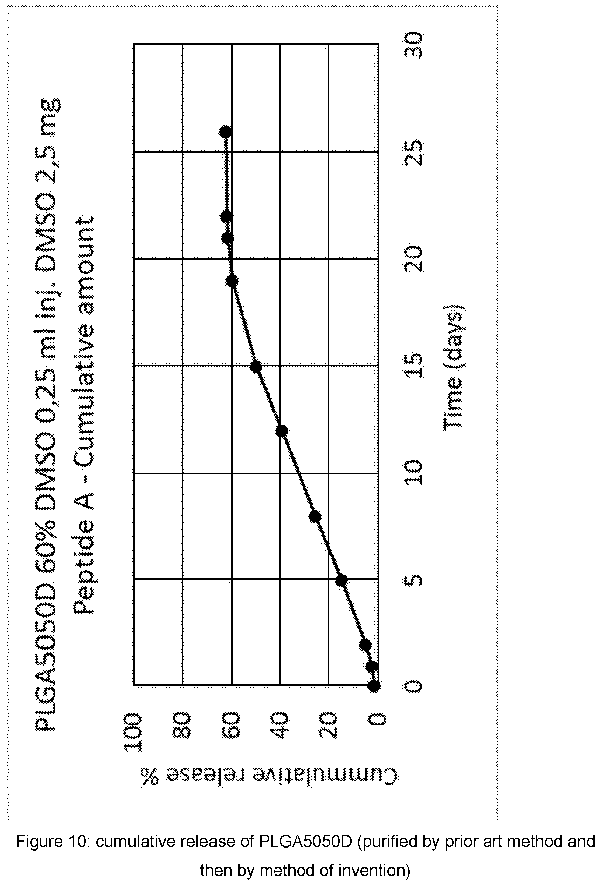

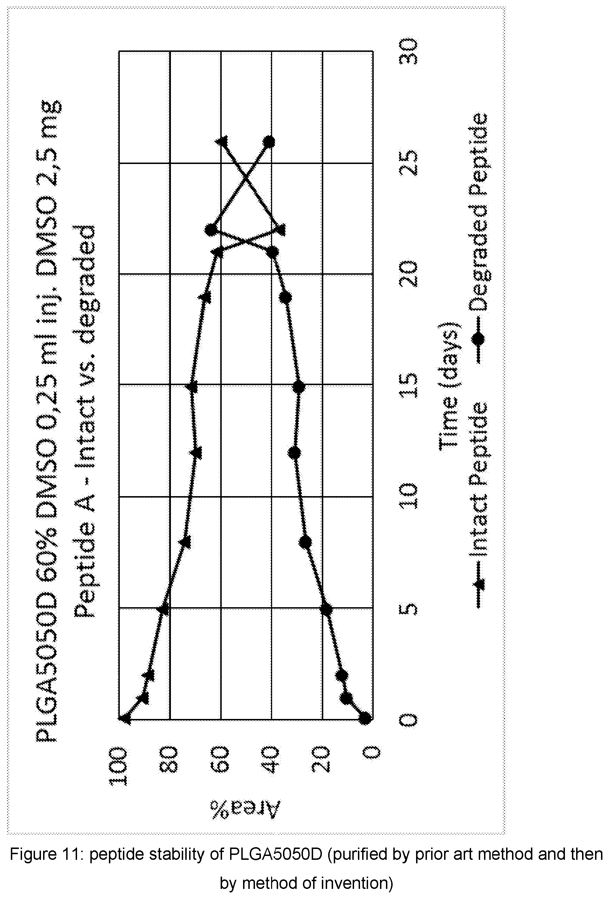

Next the drug release profiles of each solution were measured in phosphate buffered saline (PBS, pH 7.4) by injecting a predetermined amount of each solution in a stainless steel mesh screen pouch, and dropping the pouch in a PBS containing glass vessel, after which precipitation started to occur. The glass vessels were placed in a 37 degrees C tempered incubator with 60 rpm revolving speed (orbit 2 inches). The in vitro analysis of the peptide release was carried out by sampling the PBS and analysing the intact peptide A in ultra high pressure liquid chromatography equipment (UHPLC). After every sampling the PBS was replaced with fresh PBS solution. The cumulative release profiles and peptide purity graphs are shown in Figures 8-11 (Figure 8: cumulative release of PLGA5050 i.e. purified by prior art method, Figure 9: peptide stability of PLGA5050 i.e. purified by prior art method, Figure 10: cumulative release of PLGA5050D i.e. purified by prior art method and then by method of invention, Figure 11: peptide stability of PLGA5050D i.e. purified by prior art method and then by method of invention). It can be seen that the purified formulation PLGA5050D preserves the peptide A better in intact form. The final cumulative released amounts being 22 and 62 % for PLGA5050 and PLGA5050D, respectively.

### Example 4

The purification method of the invention was applied to a PLGA polymer (synthesised internally by ReBio) using DMSO and IPA in a similar manner than in example 3. The purification method was then repeated. The results were compared with a polymer where the purification method had not been applied. The results are shown below. The PLGA5050 polymer had molecular weight of 8000 g/mol. The tin amount was analysed using MP-AES (microwave Plasma-Atomic Emission Spectrometry).

| **Polymer** | **No of times method applied** | **Sn(ppm) av.** | **Decrease/ %** |
|---|---|---|---|
| **PLGA5050** | 0 | 28 | 0 |
| **PLGA5050D1** | 1 | 16.7 | 40.35714 |
| **PLGA5050D2** | 2 | 10.7 | 35.92814 |

### Example 5

The purification method of the invention was applied to a PLGA polymer using DMSO and IPA in a similar manner than in example 3. The purification method was then repeated. The results were compared with a polymer where the purification method had not been applied. Subsequently these polymers were used to prepare formulations that were studied with respect to peptide stability in the formulation and during dissolution, in the same manner as for example 2 (37 C, PBS pH 7.4, 55 % DMSO, 0, 25 ml injection). The results are shown below.

| | | | **Formul.** | **Formul.** | | |
|---|---|---|---|---|---|---|
| | **DMSO/IPA** | **Peptide** | **0 day** | **1 week** | **Dissol. 1d** | **Dissol. 8 d** |
| **Polymer** | **Wash times** | **mg/ml** | **Assay.%** | **Assay. %** | **purity-%** | **purity-%** |
| PLGA5050_0D | 0 | 10 | 56 | 0 | 77 | 35 |
| PLGA5050_1D | 1 | 10 | 83 | 1.1 | 98 | 74 |
| PLGA5050_2D | 2 | 10 | 92 | 8.8 | 96 | 87 |

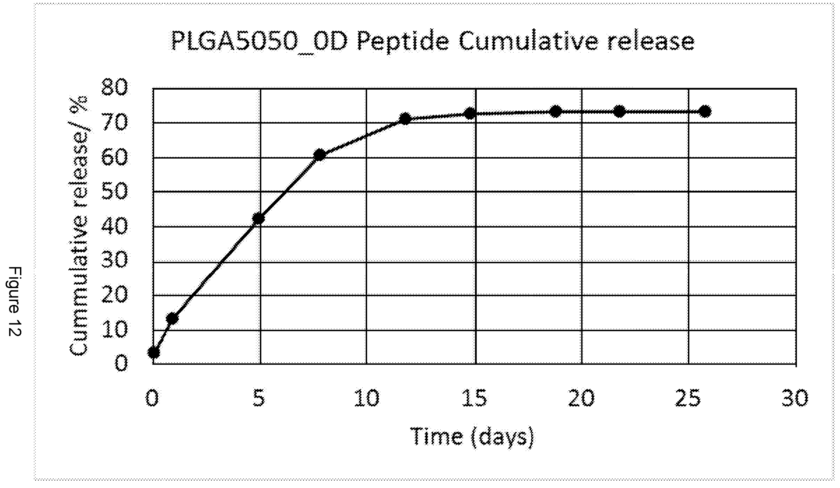

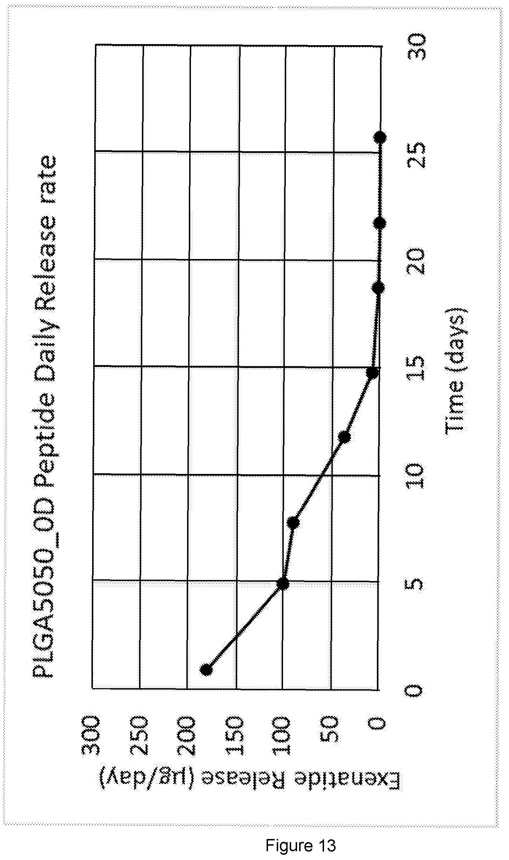

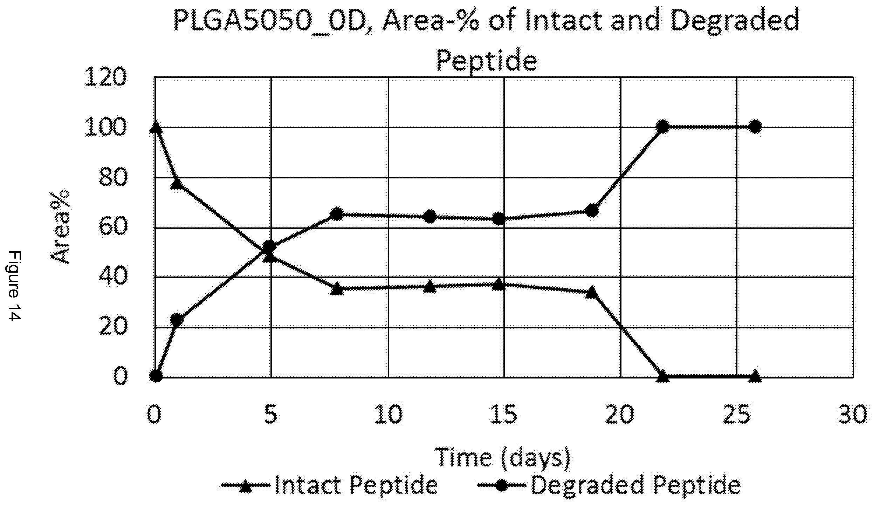

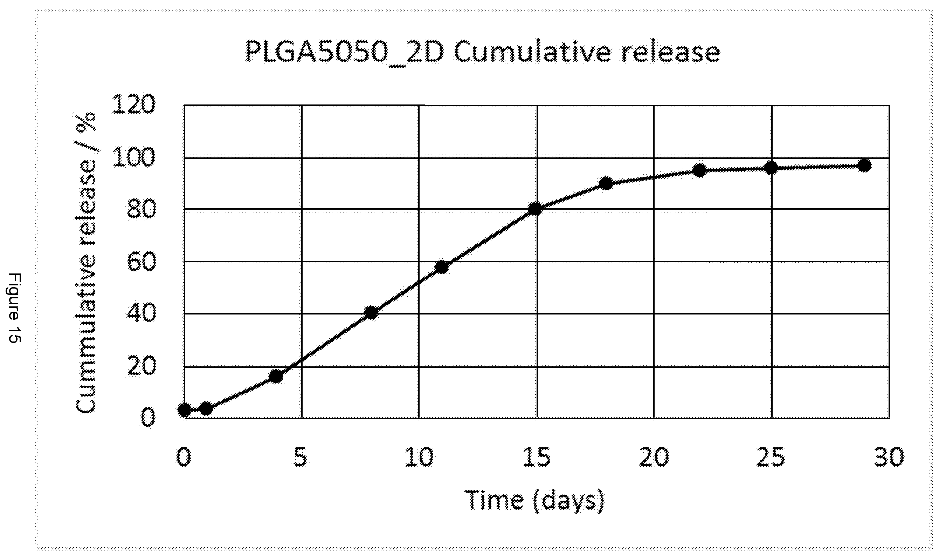

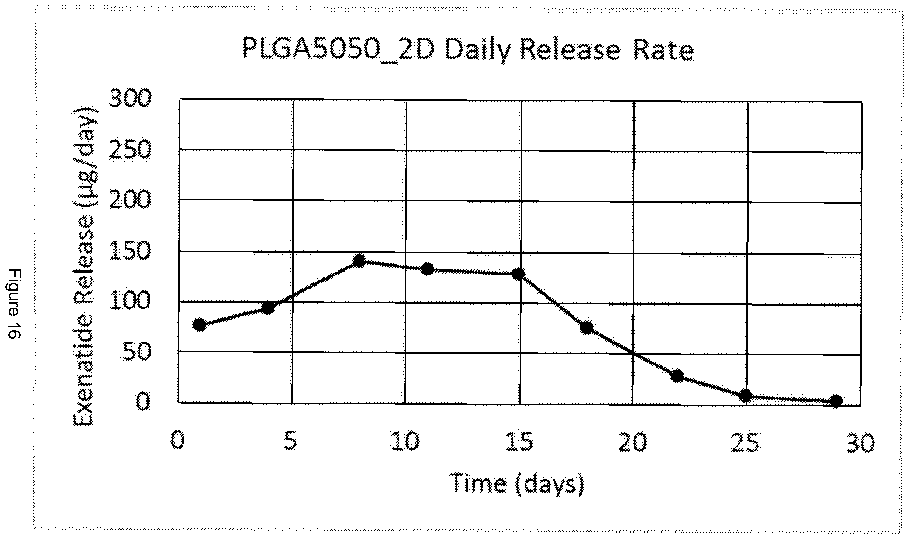

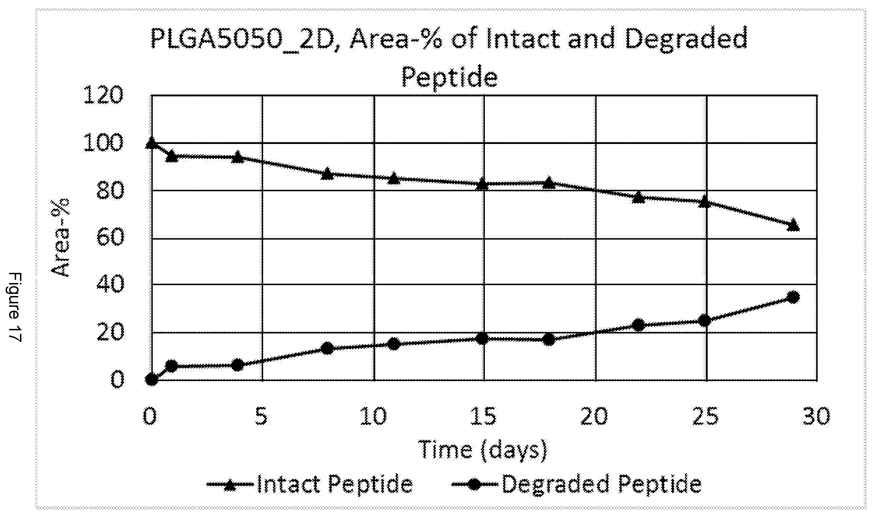

Figures 12-17 show the cumulative and daily releases and the peptide stability for each of polymers PLGA5050_0D and PLGA5050_2D (Figure 12: cumulative release for PLGA5050_0D, Figure 13: daily release for PLGA5050_0D, Figure 14: peptide stability for PLGA5050_0D, Figure 15: cumulative release for PLGA5050_2D, Figure 16: daily release for PLGA5050_2D, Figure 17: peptide stability for PLGA5050_2D). The cumulative and daily releases of intact peptide show steadier release for the 2 times washed polymer formulation (i.e. where the method of the invention has been applied twice). During dissolution, a greater proportion of the peptide degrades in the unpurified polymer formulation in addition to the fact that already at the 0-point the peptide assay of the unpurified polymer formulation is 36 % less.

### Example 6

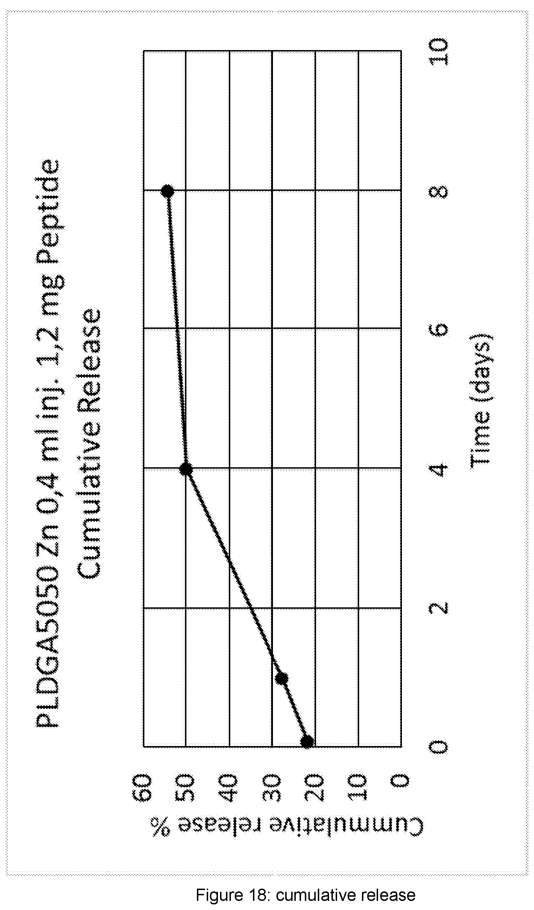

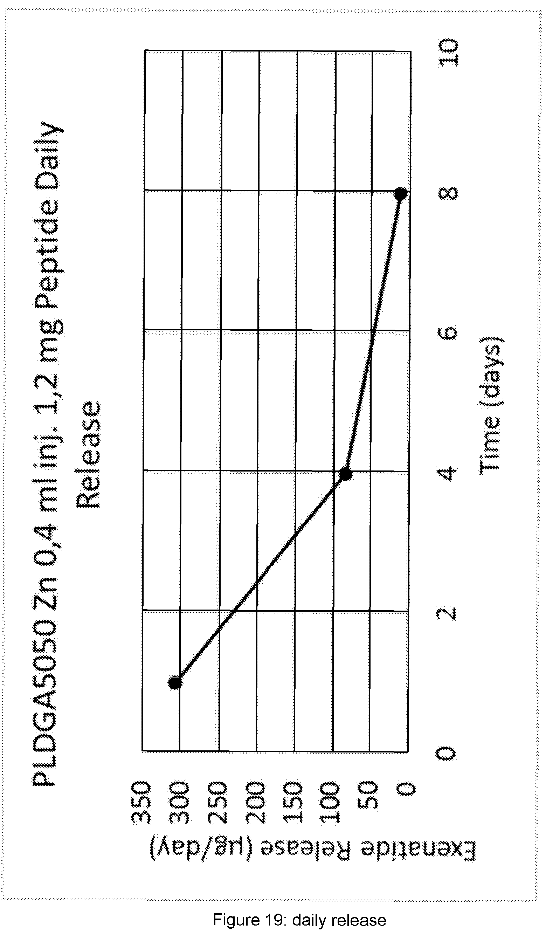

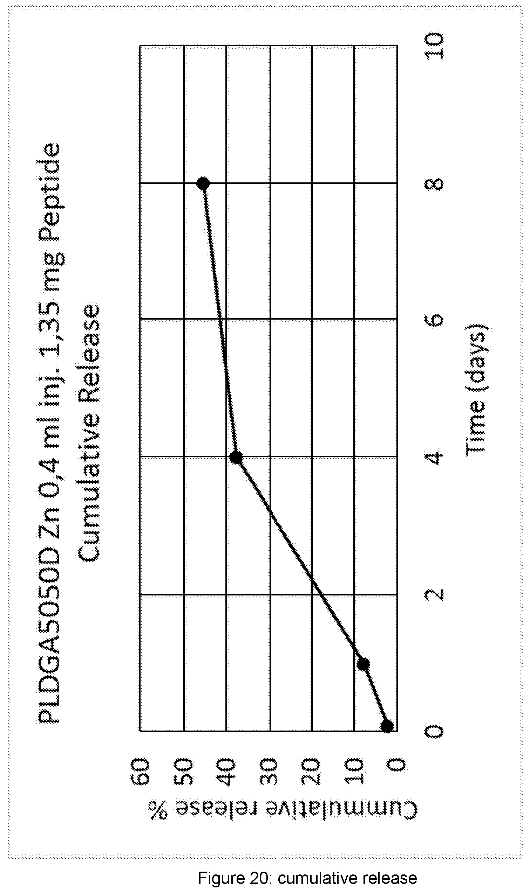

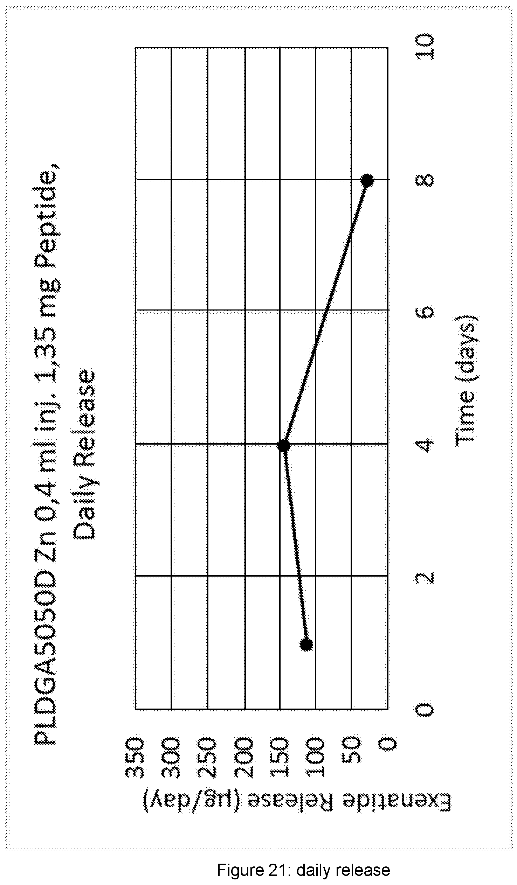

The cumulative and daily drug release from two formulations that were prepared in accordance with the methods above, using a polyester-solvent solution of 65 % DMSO and 35 % PLGA5050Zn polymer (i.e. with a non-zero Zn content) of 13 kDa. The unwashed polyester is termed PLGA5050Zn for this example and the purified polyester PLGA5050DZn. The results are shown in figures 18-21 (Figure 18: cumulative release for PLGA5050Zn, Figure 19: daily release for PLGA5050Zn, Figure 20: cumulative release for PLGA5050DZn, Figure 21: daily release for PLGA5050DZn). The unwashed PLGA5050Zn shows much higher initial drug release than once washed PLGA5050DZn as explained by cumulative drug release values of 22 and 2 % at 2 hour time point, respectively. This demonstrates the reduction in burst effect achieved by the present invention.

### Example 7

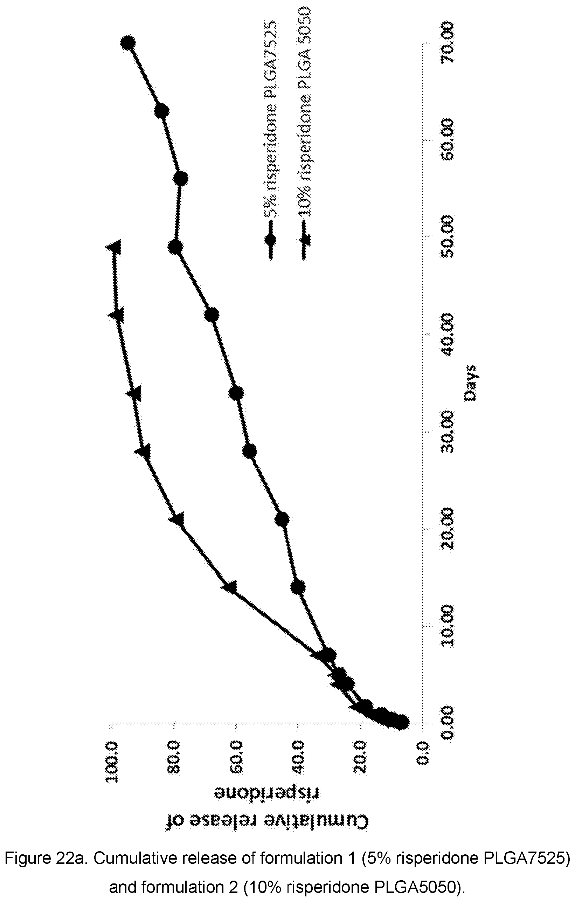

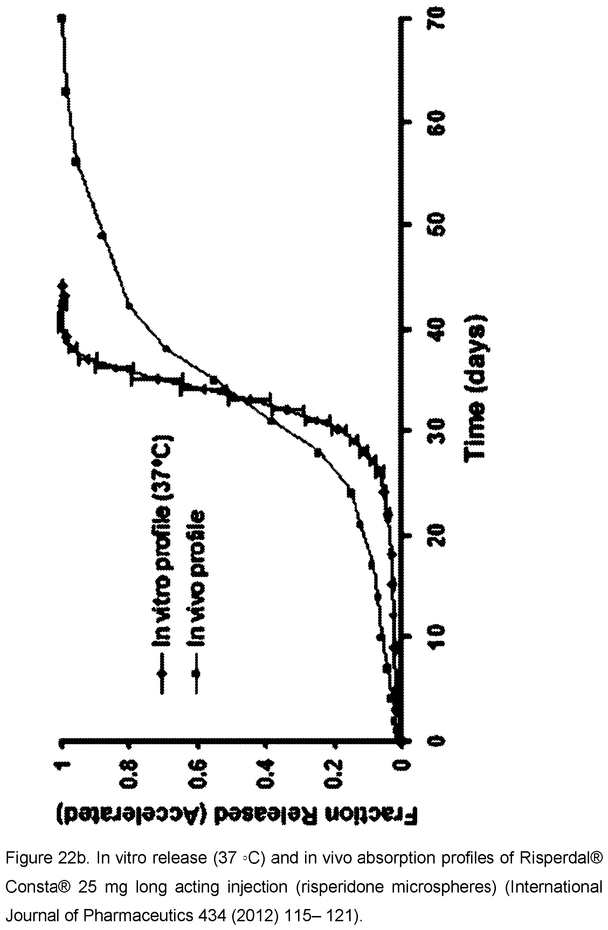

A PLGA polymer (termed PLGA5050) was purified according to the method of the invention in a similar manner to that of example 3. A further PLGA polymer (termed PLGA7525) was also purified according to the method of the invention in a similar manner to that of example 3. Each of these purified polymers were then combined with risperidone (MW = 410,485 g/mol) and NMP to form gel formulations 1 and 2. 280 mg of gel formulation 1 (35wt% PLGA7525; 5wt% risperidone; 60wt% NMP) and 140mg of gel formulation 2 (30wt% PLGA5050; 10wt% risperidone; 60wt% NMP) were injected in 500 ml PBS (pH7.4 at 37°C, 60rpm) in shaker incubator. The cumulative release of risperidone was measured using HPLC at different time points, and the results are shown in Figure 22a. Meanwhile, Figure 22b displays the cumulative release profile of a commercial microparticle risperidone product according to literature (International Journal of Pharmaceutics 434 (2012) 115- 121). Specifically, Figure 22b displays in vitro release (37 ∘C) and in vivo absorption profiles of Risperdal^{®} Consta^{®} 25 mg long acting injection (risperidone microspheres).

The comparison of Figures 22a and 22b illustrate that improved release profiles are achieved for the polymers purified according to the invention: no lag time was detected and longer release times were achieved. As such this example illustrates the improved controlled release of antipsychotic small molecule risperidone (MW = 410,485 g/mol), achieved by using the polymers purified by the present invention.

### Example 8

This example demonstrates the release of high molecular weight protein release from the purified dosage form.

A PLGA polymer (termed PLGA5050) was purified according to the method of the invention in a similar manner to that of example 3 and termed PLGA5050D. PLGA5050D was used for preparation of following formulation. PLGA5050D was dissolved in DMSO (70 %) and subsequently powder bovine serum albumin (BSA, 10 mg/ml) was suspended in the polymer solution.

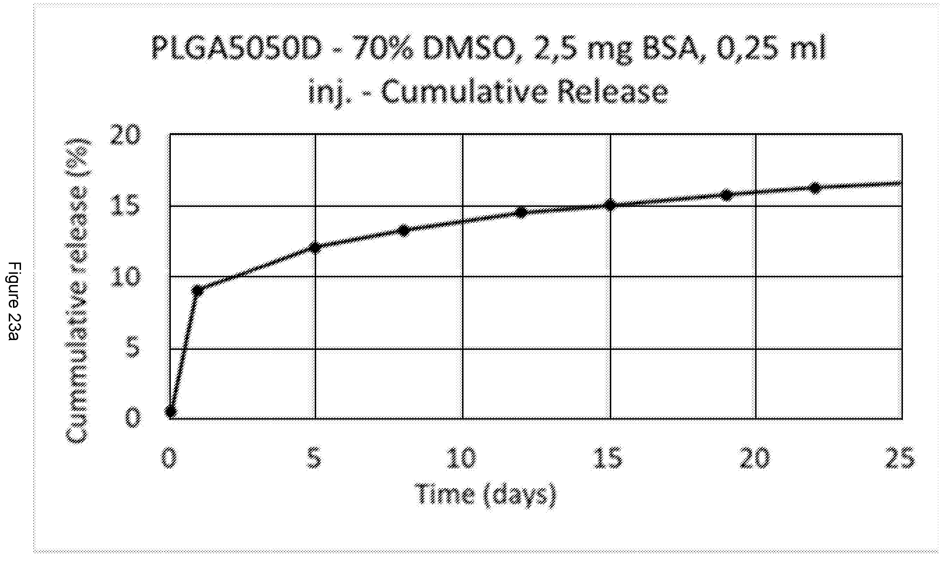

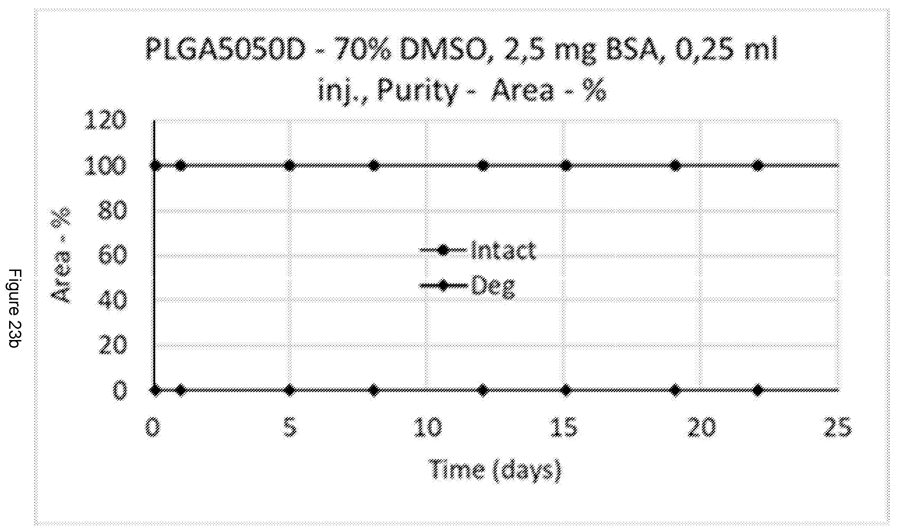

Subsequently the drug release was measured in phosphate buffered saline (PBS, pH 7.4) by injecting 0,25 ml of suspension in a stainless steel mesh screen pouch, and dropping the pouch in a PBS (12 ml) containing glass vessel, after which precipitation of the formulation started. The glass vessels were placed in a 37 °C tempered incubator with 60 rpm revolving speed (orbit 2 inches). The in vitro analysis of the BSA release was carried out by sampling the PBS and analysing the intact BSA in ultra high pressure liquid chromatography equipment (UHPLC). After every sampling the PBS was replaced with fresh PBS solution. The cumulative release profiles and protein purity graphs from the drug release experiment are shown in Figure 23a and Figure 23b respectively.

### Example 9

This example demonstrates that particularly low metal contents i.e. even below 10 ppm can be achieved with the purifying method.

Two samples of PLGA5050 were obtained, each having a Sn content of 50 ppm. These two samples of PLGA5050 were purified in a similar manner as that given in example 1, with an extended time period where the polyester was in contact with the DMSO solvent; specifically, the polyester-solvent solution was allowed to stand for 16 hours. Once the purification process was complete, the Sn content of each sample of PLGA5050 (termed PLGA5050.1 and PLGA5050.2 respectively) was measured as in Example 1. The results are shown below. These results demonstrate that the Sn removal is more pronounced when the polyester-solvent solution is allowed to stand before carrying out the precipitation step.

| | | **Sn/** | |
|---|---|---|---|
| **Polymer** | **Purification** | **ppm** | **SD** |
| PLGA5050.1 | DMSO/IPA | **5** | +1 |
| PLGA5050.2 | DMSO/IPA | **7** | ±1 |

### Example 10

This example demonstrates the final product usage as part of a kit, more specifically. The kit of the present example enables a controlled release pharmaceutical composition in the form of an in-situ forming implant, where the kit packaging is filled, the product mixed, the needle attached, and the composition injected subcutaneously.

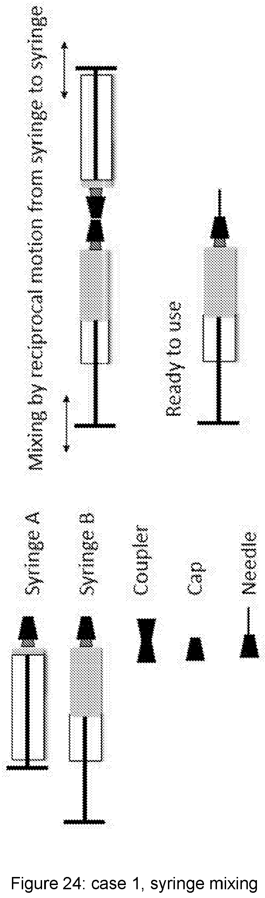

### Case 1: kit provided as a syringe mixing kit (figure 24)

This product must be at room temperature prior to injection. The kit is provided in the form of sterile part A and B in two syringes (figure 24). Part A comprises the weighed drug in the powder form in the tip of syringe A which is capped. Part B comprises the excipient purified bioresorbable polyester and solvent in a fluid form in another syringe B which is also capped. The caps are removed from the syringes and then they are combined with small sterile coupler (however, another option would be to use syringes that can be coupled straight, e.g syringes with male and female luer-lock). Mixing is carried out by compressing the fluid from syringe B through the coupler to the syringe A that contains the drug. Then, the formed mixture of drug and fluid is compressed again from syringe A to the syringe B. This reciprocal movement is repeated 20 times and drug suspension is left to syringe A such that the drug is homogenously suspended in the fluid in the syringe A. The syringe A is detached from the coupler and the hypodermic needle inserted to the tip. Prior to administration, the syringe is purged in upright position to remove any large air bubbles from the needle. The dosage is injected e.g subcutaneously to the fatty tissue to the desired place such as back of upper arm, stomach, buttocks or thigh. After injection the needle is kept in its place for approximately a few seconds before removing it from tissue.

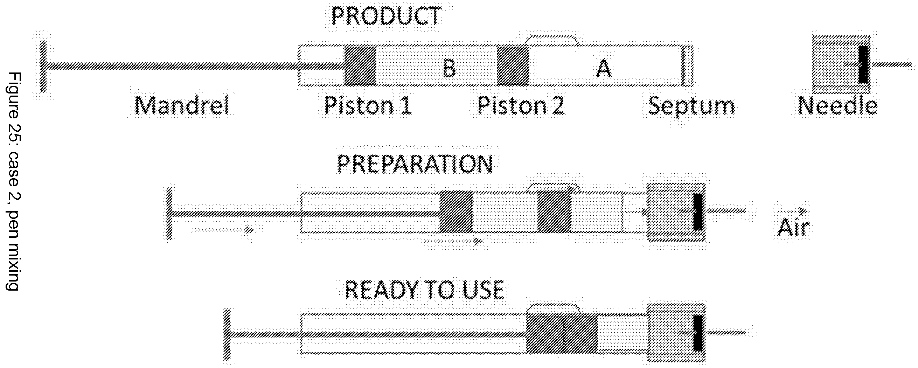

### Case 2: kit provided as a pen mixing kit. (figure 25)

This product must be at room temperature prior to injection. The kit is provided in the form of pen that comprises 2 syringe pistons, mandrel, septum, needle with inner thread and transparent cylinder with side runner channel(s) holding pistons inside as shown in figure 25. The solid drug powder is stored in compartment A and purified bioresorbable polyester and solvent in a fluid form in compartment B separated by the pistons. Before administration the following steps are carried out. The needle is tightened to the septum while the inner part of needle punctures the septum. Then the mandrel is pushed forward in upright position which makes the pistons 1 and 2 move with the fluid. Piston 2 stops moving when it reaches the position at the side runner channel(s) while piston 1 continues moving pushing the fluid from compartment B to compartment A through the side channel(s). Air from compartment A may escape from the needle. The mandrel is pushed forward until piston 1 reaches piston 2, at which point it stops; the drug and fluid are now combined in compartment A. Next the drug is mixed with fluid by tapping it against other hand holding from cylinder until the mixture is homogenous - the tapping time can be selected depending on the tapping rate/speed. Prior to administration, the mandrel is turned clockwise 90° when further movement of the mandrel is allowed. The pend should be purged in upright position to remove any air from the needle. The dosage is injected e.g subcutaneously to the fatty tissue to the desired place such as back of upper arm, stomach, buttocks or thigh. After injection, the needle is kept in place approximately for few seconds before removing it from tissue.

### Example 11

This example demonstrates the reduction in residual monomer content of a polyester achieved by subjecting the polyester to the purifying method. The residual monomer content of a PLGA polymer that had not been subjected to the purifying method disclosed herein (PLGA5050_CP) was measured. The residual monomer content of two PLGA polymers that had been subjected to the purifying method disclosed herein (PLGA5050_W1a, PLGA5050_W1b) was also measured. Finally, the residual monomer content of a PLGA polymer that had been subjected to the purifying method disclosed herein twice (PLGA5050_2xW) was measured. The results are shown below.

| | **Glycolide** | **Lactide** | **Total** |
|---|---|---|---|
| **Sample** | **wt.%** | **wt.%** | **wt.%** |
| PLGA5050_CP | 0.62 | 1.4 | 2.02 |
| PLGA5050_W1a | 0.07 | 0.23 | 0.29 |
| PLGA5050_W1b | 0.05 | 0.24 | 0.29 |
| PLGA5050_2xW | <LOQ* | <LOQ | NA |
| *limit of quantification = 0.005 wt% | | | |

### Example 12

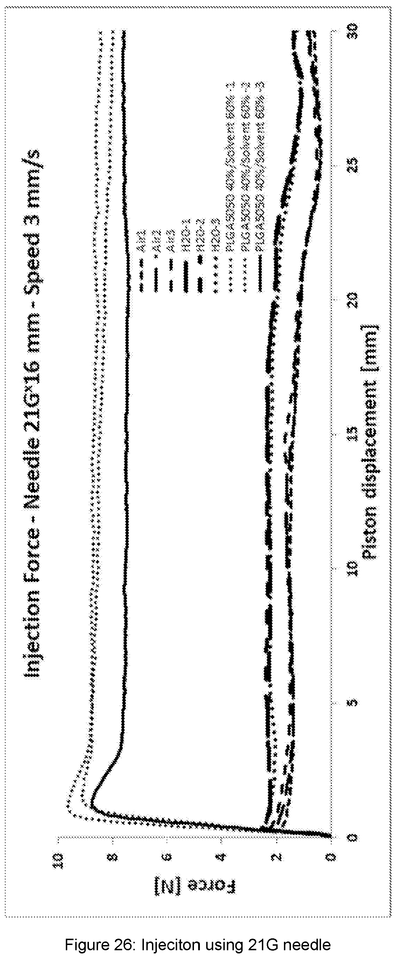

This example demonstrates the ease with which the formulation can be used by way of injection, indicating that the formulation has an appropriate viscosity for injection. An injection force though a 21G needle was used. Test setup was a 200 N cell. A polyethylene 1 ml syringe was used, with a rubber seal in plunger, a 21 G needle, and L16 mm. Compositions using 60% of purified PLGA (PLGA5050) in 60% DMSO solvent were injected. Injection of water and air were used as references. Results are shown in Figure 26 and have a good thumb force level < 10 N and express good reproducibility.
A separate practical test was also done by hand with a 23G needle, which also showed good thumb level force.

### Example 13

This example demonstrates the stability of a peptide when used with a polymer that has been purified using the method disclosed herein.

Peptide degradation was measured for a formulation containing 44% of unpurified PLGA polymer (PLGA5050_CP), 55% of DMSO and 1 % of peptide. Peptide degradation was also measured for corresponding formulations that instead contained either a PLGA polymer subjected once to the purifying method disclosed herein (PLGA5050_1xW) or a PLGA subjected twice to the purifying method disclosed herein (PLGA5050_2xW). The results are shown in the table below:

| | Assay/ |
|---|---|
| Sample | Area-% |
| PLGA5050_CP | 74.0 |
| PLGA5050_1xW | 98.9 |
| PLGA5050_2xW | 100 |

Subsequently the stability of the peptide in the formulations containing either PLGA5050_1xW or PLGA5050_2xW were monitored over extended periods of time. The results are shown in the table below:

| | Peptide formulation assay/ Area-% | | |
|---|---|---|---|
| Sample | 0 h | 4 h | 20 h |
| PLGA5050_1xW | 100 | 88 | 55.7 |
| PLGA5050_2xW | 100 | 99.4 | 95.9 |

### Example 14

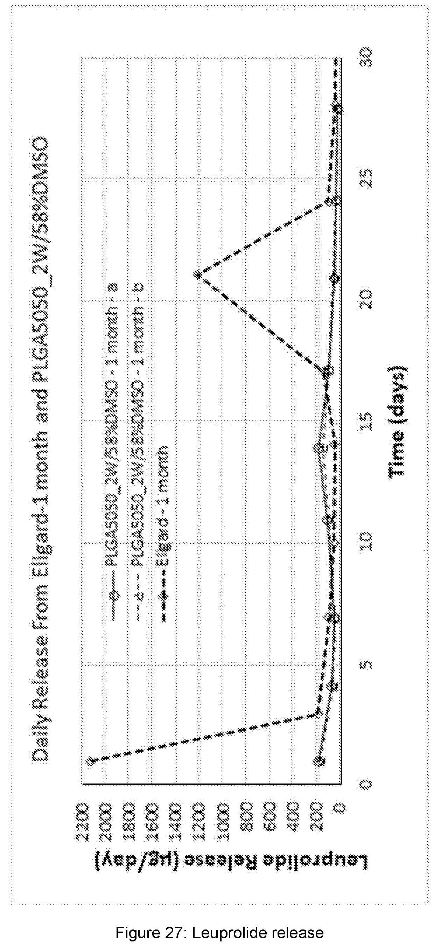

This example demonstrates the improvement of the drug release profile afforded by the compositions purified according to the method disclosed herein in comparison to a commercially available product, the Eligard-1 month product. A polymer that had been subjected to the purifying method disclosed herein twice (PLGA5050_2xW) was dissolved in DMSO, such that the resulting formulation contained 58 % DMSO and 42 % PLGA5050_2xW. Subsequently 10 mg/ml of lyophilised Leuprolide acetate was suspended in the formulation. Next 0,25 ml (2,5 mg Leuprolide acetate) of this formulation (containing leuprolide acetate) was injected into a dissolution medium and the release of the drug was measured for 30 days (2 parallels) in a similar manner to example 8. The results were compared to a corresponding study that was carried out for the Eligard-1 month product (7,5 mg Leuprolide acetate). The daily drug release curves are shown in Figure 27. The release from the formulation containing the purified polymer was without primary and secondary burst, whilst such bursts were observed for the Eligard-1 month product (see the release curve at days 1 and 21). The PLGA5050_2W(42%)/DMSO(58%) formulation gave a steady drug release from the beginning to the end of the 30 day dissolution period with similar nominal release with Eligard 1 month.

## Claims

1. A controlled release pharmaceutical composition comprising at least one active pharmaceutical ingredient in a biocompatible solvent and at least one polyester, wherein the pharmaceutical composition is in the form of an in-situ forming implant composition, wherein the biocompatible solvent is selected from N-methyl-2-pyrrolidone, triacetin, dimethylsulfoxide, benzyl benzoate, glycofurol or combinations thereof, wherein the polyester is a bioresorbable polyester and wherein the metal content of the polyester is > 0 ppm and < 40ppm.

2. The controlled release pharmaceutical composition according to claim 1, wherein the active pharmaceutical ingredient includes one or more H2 receptor antagonists, antimuscarinics, prostaglandin analogues, non-steroidal anti-inflammatory agents, proton pump inhibitors, aminosalycilates, corticosteroids, chelating agents, cardiac glycosides, phosphodiesterase inhibitors, thiazide, diuretics, anesthetic agents, carbonic anhydrase inhibitors, antihypertensives, anti-cancers, anti-depressants, calcium channel blockers, analgesics, opioid antagonists, antiplatels, anticoagulants, fibrinolytics, statins, adrenoceptor agonists, beta blockers, antihistamines, respiratory stimulants, micolytics, expectorants, barbiturates, anxiolytics, central nervous system agents, tricyclic antidepressants, 5HT1 antagonists, opiates, 5HT1 agonists, antiemetics, antiepileptics, dopaminergics, antibiotics, antifungals, anthelmintics, antivirals, antiprotozoals, antidiabetics, insulin and its derivatives, GLP-1 agonists, thyrotoxins, female sex hormones, male sex hormones, antioestrogens, hypothalamics, pituitary hormones, posterior pituitary hormone antagonists, peptide drugs, protein drugs, protein kinases, antigens, antidiuretic hormone antagonists, bisphosphonates, dopamine receptor stimulants, androgens, steroid reductase inhibitors, non-steroidal anti-inflammatories, immuno suppressants, local anaesthetic, sedatives, anti-psoriatics, silver salts, topical antibacterials, vaccines, or vaccine antigens;
preferably wherein the at least one active pharmaceutical ingredient includes one or more GLP-1 receptor agonists selected from one or more of exenatide, liraglutide, lixisenatide, albiglutide, dulaglutide, taspoglutide, and semaglutide, or one or more central nervous system agents selected from one or more of risperidone, olanzapine, quetiapine, ziprasidone , aripiprazole, paliperidone, haloperidol, lurasidone, fluoxetine, citalopram, sertraline, paroxetine, escitalopram, clonazepam, alprazolam, lorazepam, carbamazepine, lamotrigine, and oxcarbazepine.

3. A method of forming a controlled release pharmaceutical composition, wherein the pharmaceutical composition is in the form of an in-situ forming implant composition, comprising purifying a bioresorbable polyester with a metal content of > 0 ppm comprising the steps of:
i) dissolving the polyester in an organic solvent to form a polyester-solvent solution, wherein the organic solvent comprises at least one heteroatom selected from oxygen, nitrogen, sulphur and phosphorus;
ii) precipitating the polyester from the polyester-solvent solution by combining the polyester-solvent solution with an organic non-solvent, said non-solvent being an alcohol; and
(iii) separating the precipitated polyester from the solvent and non-solvent to obtain a purified polyester;
such that the metal content of the purified polyester is > 0 ppm and < 40ppm; and
(iv)combining the purified polyester with at least one active pharmaceutical ingredient in a biocompatible solvent;
to form the controlled release composition, wherein the biocompatible solvent is selected from N-methyl-2-pyrrolidone, triacetin, dimethylsulfoxide, benzyl benzoate, glycofurol or combinations thereof.

4. A kit for injecting a subject with a controlled release pharmaceutical composition, wherein the pharmaceutical composition is in the form of an in-situ forming implant composition, comprising,
a first container comprising at least one bioresorbable polyester with a metal content of > 0 ppm and < 40ppm, and a biocompatible solvent selected from N-methyl-2-pyrrolidone, triacetin, dimethylsulfoxide, benzyl benzoate, glycofurol or combinations thereof;
a second container comprising at least one active pharmaceutical ingredient; and a needle capable of attachment to at least one of the first or the second container.

5. The kit according to claim 4, wherein the active pharmaceutical ingredient is according to claim 2.

6. The kit according to claims 4 or 5, wherein the active pharmaceutical ingredient is provided as a desiccated powder.

7. The kit according to any of claims 4 to 6, wherein the first and second containers are either provided as separate parts that are capable of being connected together, or as two integrally formed compartments.

8. The kit according to any of claims 4 to 7, further comprising instructions for use, wherein the instructions for use require the mixing of the contents of the first and second container, following by the injection of the resulting mixture into a subject, preferably wherein the kit further comprises a means for mixing the contents of the first container with the contents of the second container, and a means for agitating the resulting mixture.

## Patentansprüche

1. Pharmazeutische Zusammensetzung mit kontrollierter Freigabe, die mindestens einen pharmazeutischen Wirkstoff in einem biokompatiblen Lösungsmittel und mindestens ein Polyester umfasst, wobei die pharmazeutische Zusammensetzung in der Form einer vor Ort bildenden Implantatzusammensetzung ist, wobei das biokompatible Lösungsmittel ausgewählt ist aus N-Methyl-2-pyrrolidon, Triacetin, Dimethylsulfoxid, Benzylbenzoat, Glycofurol oder Kombinationen davon, wobei der Polyester ein bioresorbierbarer Polyester ist, und wobei der Metallgehalt des Polyesters > 0 ppm und < 40 ppm beträgt.

2. Pharmazeutische Zusammensetzung mit kontrollierter Freigabe nach Anspruch 1, wobei der pharmazeutische Wirkstoff einen oder mehrere H2-Rezeptorantagonisten, Antimuskarinika, Prostaglandinanaloge, nichtsteroidale entzündungshemmende Mittel, Protonenpumpenhemmer, Aminosalicylate, Kortikosteroide, Chelatbildner, Herzglykoside, Phosphodiesterasehemmer, Thiazid, Diuretika, Anästhetika, Carboanhydrasehemmer, Blutdrucksenker, Krebsmittel, Antidepressiva, Calciumkanalblocker, Analgetika, Opioid-Antagonisten, Plättchenhemmer, Antikoagulanse, Fibrinolytika, Statine, Adrenorezeptoragonisten, Betablocker, Antihistaminika, Atemstimulanzien, Mukolytika, Schleimlöser, Barbiturate, Anxiolytika, Mittel für das Zentralnervensystem, trizyklische Antidepressiva, 5HT1-Antagonistem, Opiate, 5HT1-Agonisten, Antiemetika, Antiepileptika, dopaminerge Mittel, Antibiotika, Antimykotika, Anthelminthika, antivirale Mittel, Antiprotozoika, Antidiabetika, Insulin und dessen Derivate, GLP-1-Agonisten, Thyrotoxine, weibliche Geschlechtshormone, männliche Geschlechtshormone, Östrogenantagonisten, hypothalamische Mittel, Hypophysen-Hormone, Antagonisten der Hormone des Hinterlappens der Hypophyse, Peptid-Arzneimittel, Protein-Arzneimittel, Proteinkinasen, Antigene, Antagonisten des antidiuretischen Hormons, Bisphosphonate, Dopaminerezeptorstimulanzien, Androgene, Steroidreduktasehemmer, nichtsteroidale Entzündungshemmer, Immunsuppresiva, lokale Anästhetika, Sedativa, Antipsoriatika, Silbersalze, topische antibakterielle Mittel, Impfungen oder Impfungsantigene einschließt; wobei der mindestens eine pharmazeutische Wirkstoff vorzugsweise einen oder mehrere GLP-1-Rezeptoragonisten einschließt, ausgewählt aus einem oder mehreren von Exenatid, Liraglutid, Lixisenatid, Albiglutid, Dulaglutid, Taspoglutid und Semaglutid, oder einem oder mehreren Mitteln für das Zentralnervensystem ausgewählt aus einem oder mehreren von Risperidon, Olanzapin, Quetiapin, Ziprasidon, Aripiprazol, Paliperidon, Haloperidol, Lurasidon, Fluoxetin, Citalopram, Sertralin, Paroxetin, Escitalopram, Clonazepam, Alprazolam, Lorazepam, Carbamazepin, Lamotrigin und Oxcarbazepin.

3. Verfahren zum Bilden einer pharmazeutischen Zusammensetzung mit kontrollierter Freigabe, wobei die pharmazeutische Zusammensetzung in der Form einer vor Ort bildenden Implantatzusammensetzung ist, und Reinigen eines bioresorbierbaren Polyesters mit einem Metallgehalt von > 0 ppm umfasst, umfassend die folgenden Schritte:
i) Auflösen des Polyesters in einem organischen Lösungsmittel, um eine Polyester-Lösungsmittel-Lösung zu bilden, wobei das organische Lösungsmittel mindestens ein Heteroatom umfasst, ausgewählt aus Sauerstoff, Stickstoff, Schwefel und Phosphor;
ii) Ausfällen des Polyesters aus der Polyester-Lösungsmittel-Lösung durch Kombinieren der Polyester-Lösungsmittel-Lösung mit einem organischen Nichtlösungsmittel, wobei das Nichtlösungsmittel ein Alkohol ist; und
(iii) Trennen des ausgefällten Polyesters aus dem Lösungsmittel und Nichtlösungsmittel, um einen gereinigten Polyester zu erhalten;
sodass der Metallgehalt des gereinigten Polyesters > 0 ppm und < 40ppm beträgt; und
(iv) Kombinieren des gereinigten Polyesters mit mindestens einem pharmazeutischen Wirkstoff in einem biokompatiblen Lösungsmittel;
um die Zusammensetzung mit kontrollierter Freigabe zu bilden, wobei das biokompatible Lösungsmittel ausgewählt ist aus N-Methyl-2-pyrrolidon, Triacetin, Dimethylsulfoxid, Benzylbenzoat, Glycofurol oder Kombinationen davon.

4. Kit zum Injizieren einer pharmazeutischen Zusammensetzung mit kontrollierter Freigabe in ein Subjekt, wobei die pharmazeutische Zusammensetzung in der Form einer vor Ort bildenden Implantatzusammensetzung ist, umfassend,
einen ersten Behälter, der mindestens einen bioresorbierbaren Polyester mit einem Metallgehalt von > 0 ppm und < 40 ppm umfasst, und ein biokompatibles Lösungsmittel, ausgewählt aus N-Methyl-2-pyrrolidon, Triacetin, Dimethylsulfoxid, Benzylbenzoat, Glycofurol oder Kombinationen davon;
einen zweiten Behälter, der mindestens einen pharmazeutischen Wirkstoff umfasst; und eine Nadel, die fähig ist zur Befestigung an mindestens einem des ersten oder zweiten Behälters.

5. Kit nach Anspruch 4, wobei der pharmazeutische Wirkstoff nach Anspruch 2 ist.

6. Kit nach Anspruch 4 oder 5, wobei der pharmazeutische Wirkstoff als ein getrocknetes Pulver bereitgestellt ist.

7. Kit nach einem der Ansprüche 4 bis 6, wobei der erste und der zweite Behälter entweder als getrennte Teile bereitgestellt sind, die fähig sind, untereinander verbunden zu werden, oder als zwei einstückig geformte Kammern.

8. Kit nach einem der Ansprüche 4 bis 7, weiter umfassend Verwendungsanweisungen, wobei die Verwendungsanweisungen das Mischen des Inhalts des ersten und des zweiten Behälters gefolgt von Injektion der resultierenden Mischung in ein Subjekt erforderlich machen, wobei das Kit vorzugsweise weiter ein Mittel zum Mischen des Inhalts des ersten und des zweiten Behälters und ein Mittel zum Schütteln der resultierenden Mischung umfasst.

## Revendications

1. Composition pharmaceutique à libération contrôlée comprenant au moins un principe actif pharmaceutique dans un solvant biocompatible et au moins un polyester, dans laquelle la composition pharmaceutique se présente sous la forme d'une composition d'implant se formant sur site, dans laquelle le solvant biocompatible est sélectionné à partir de la N-méthyl-2-pyrrolidone, de la triacétine, du diméthylsulfoxyde, du benzoate de benzyle, du glycofurol ou des combinaisons de ceux-ci, dans laquelle le polyester est un polyester biorésorbable et dans laquelle la teneur en métal du polyester est > 0 ppm et < 40 ppm.

2. Composition pharmaceutique à libération contrôlée selon la revendication 1, dans laquelle le principe actif pharmaceutique inclut un ou plusieurs antagonistes des récepteurs H2, des antimuscariniques, des analogues de la prostaglandine, des agents anti-inflammatoires non stéroïdiens, des inhibiteurs de la pompe à protons, des amynosalycilates, des corticostéroïdes, des agents chélateurs, des glycosides cardiaques, des inhibiteurs de la phosphodiestérase, du thiazide, des diurétiques, des agents anesthésiques, des inhibiteurs de l'anhydrase carbonique, des antihypertenseurs, des agents anticancéreux, des antidépresseurs, des bloqueurs des canaux calciques, des antalgiques, des antagonistes des opioïdes, des agents antiplaquettaires, des anticoagulants, des fibrinolytiques, des statines, des agonistes des adrénorécepteurs, des bêtabloquants, des antihistaminiques, des stimulants respiratoires, des mucolytiques, des expectorants, des barbituriques, des anxiolytiques, des agents du système nerveux central, des antidépresseurs tricycliques, des antagonistes de 5HT1, des opioïdes, des agonistes de 5HT1, des antiémétiques, des antiépileptiques, des dopaminergiques, des antibiotiques, des antifongiques, des antihelminthiques, des antiviraux, des antiprotozoaires, des antidiabétiques, de l'insuline et de ses dérivés, des agonistes de GLP-1, des thyrotoxines, des hormones sexuelles féminines, des hormones sexuelles masculines, des anticestrogènes, des hypothalamiques, des hormones pituitaires, des antagonistes des hormones pituitaires postérieures, des médicaments peptidiques, des médicaments protéiques, des protéine-kinases, des antigènes, des antagonistes des hormones antidiurétiques, des biphosphonates, des stimulateurs de récepteurs de la dopamine, des androgènes, des inhibiteurs des réductases stéroïdiennes, des anti-inflammatoires non stéroïdiens, des immunosuppresseurs, des anesthésiques locaux, des sédatifs, des anti-psoriasiques, des sels d'argent, des antibactériens topiques, des vaccins ou des antigènes vaccinaux ;
de préférence dans laquelle l'au moins un principe actif pharmaceutique inclut un ou plusieurs agonistes des récepteurs de GLP-1 sélectionnés parmi un ou plusieurs parmi l'exénatide, le liraglutide, le lixisénatide, l'albiglutide, le dulaglutide, le taspoglutide et le sémaglutide ou un ou plusieurs agents du système nerveux central sélectionnés à partir d'un ou plusieurs parmi la rispéridone, l'olanzapine, la quétiapine, la ziprasidone, l'aripiprazole, la palipéridone, l'halopéridol, la lurasidone, la fluoxétine, le citalopram, la sertraline, la paroxétine, l'escitalopram, le clonazépam, l'alprazolam, le lorazépam, la carbamazépine, la lamotrigine et l'oxcarbazépine.

3. Procédé de formation d'une composition pharmaceutique à libération contrôlée, dans lequel la composition pharmaceutique se présente sous la forme d'une composition d'implant se formant sur site, comprenant la purification d'un polyester biorésorbable avec une teneur en métal > 0 ppm comprenant les étapes de :
i) la dissolution du polyester dans un solvant organique pour former une solution de polyester-solvant, dans laquelle le solvant organique comprend au moins un hétéroatome sélectionné parmi l'oxygène, l'azote, le soufre et le phosphore ;
ii) la précipitation du polyester de la solution de polyester-solvant en combinant la solution de polyester-solvant avec un non-solvant organique, ledit non-solvant étant un alcool ; et
iii) la séparation du polyester précipité du solvant et d'un non-solvant pour obtenir un polyester purifié ;
de telle sorte que la teneur en métal du polyester purifié est > 0 ppm et < 40 ppm ; et
iv) la combinaison du polyester purifié avec au moins un principe actif pharmaceutique dans un solvant biocompatible ;
pour former la composition à libération contrôlée, dans laquelle le solvant biocompatible est sélectionné parmi la N-méthyl-2-pyrrolidone, la triacétine, le diméthylsulfoxide, le benzoate de benzyle, le glycofurol ou des combinaisons de ceux-ci.

4. Kit pour l'injection à un sujet d'une composition pharmaceutique à libération contrôlée, dans lequel la composition pharmaceutique se présente sous la forme d'une composition d'implant se formant sur site, comprenant,
un premier récipient comprenant au moins un polyester biorésorbable avec une teneur en métal > 0 ppm et < 40 ppm et un solvant biocompatible sélectionné parmi la N-méthyl-2-pyrrolidone, la triacétine, le diméthylsulfoxyde, le benzoate de benzyle, le glycofurol ou des combinaisons de ceux-ci ;
un second récipient comprenant au moins un principe actif pharmaceutique ; et une aiguille capable de se fixer à au moins l'un du premier ou du second récipient.

5. Kit selon la revendication 4, dans lequel le principe actif pharmaceutique est selon la revendication 2.

6. Kit selon la revendication 4 ou la revendication 5, dans lequel le principe actif pharmaceutique est fourni en tant que poudre desséchée.

7. Kit selon l'une quelconque des revendications 4 à 6, dans lequel les premier et second récipients sont soit fournis en tant qu'éléments séparés qui sont capables d'être reliés ensemble, soit en tant que deux compartiments formés d'un seul tenant.

8. Kit selon l'une quelconque des revendications 4 à 7, comprenant en outre des instructions d'utilisation, dans lequel les instructions d'utilisations requièrent le mélange des contenus des premier et second récipients, suivi par l'injection du mélange résultant à un sujet, de préférence dans lequel le kit comprend en outre un moyen pour le mélange du contenu du premier récipient avec le contenu du second récipient et un moyen pour l'agitation du mélange résultant.
